# EUROPEAN PATENT APPLICATION

(11) **EP 4 379 735 A1**
(43) Date of publication of application: **05.06.2024**
(21) Application number: 22210334.3
(22) Date of filing: 29.11.2022
(51) Int. Cl.: G16H 30/20, A61B 1/24, G06T 19/00, G16H 30/40, G16H 50/20, G16H 50/50

(54) **TECHNOLOGIES FOR GINGIVITIS DETECTION AND PERIODONTAL POCKET DEPTH ASSESSMENT**

(71) Applicant: 3Shape Trios A/S, 1060 Copenhagen (DK); Colgate-Palmolive Company, New York, NY 10022 (US)
(72) Inventor: SANGILD SORENSEN, Thomas, 1060 Copenhagen (DK); SCHARFE LAMBACH, Mathias, 1060 Copenhagen (DK); VANNAHME, Christoph, 1060 Copenhagen (DK); MICHOU, Stavroula, 1060 Copenhagen (DK); NORRISGAARD, Pia Elisabeth, 1060 Copenhagen (DK); HUSEINI, Admir, 1060 Copenhagen (DK); CEBOV, Aleksandar, 1060 Copenhagen (DK); CHOJNACKI, Marek, 1060 Copenhagen (DK); HOGAN, Richard, Urmston, M41 0TY (GB); ELLWOOD, Roger Philip, Anglesey, LL75 8PZ (GB)
(74) Representative: Studio Torta S.p.A.

(57) **Abstract**

Technologies are disclosed for detection and display of an indication of a gingivitis condition and/or a periodontal pocket assessment of a subject's oral cavity via a digital representation of the oral cavity. Using scan data of the oral cavity, one or more teeth in the oral cavity may be indicated. A first assessment location proximate to a first tooth of the one or more teeth may be indicated. A first image may be generated that may include the first assessment location and one or more first data channels. The one or more first data channels may comprise color data and topological information corresponding to the first assessment location. Using one or more machine-learning algorithms, a modified gingival index (MGI) value and/or a periodontal pocket depth assessment may be determined and displayed for the first assessment location based on the first image and the one or more first data channels.

## Description

### BACKGROUND

Dental tissue issues such as, for example, periodontal disease and tooth cavities are often caused by certain bacterial species in the mouth that interact with proteins present in saliva to form a film, known as plaque, that coats the teeth. If such build-up progresses, the acid produced by the bacteria can attack the teeth resulting in tooth decay. The plaque also may attack the soft gum tissue of the mouth leading to gingivitis, or periodontitis, which may affect much, if not all, of the soft tissue and bone supporting the teeth.

Gingivitis is a form of gum disease that causes material irritation of the gingiva. The gingiva is a part of the oral mucosa that covers the alveolar bone and tooth root to a level just coronal to the cement-enamel junction. In other words, the gingiva is the area/section of the gum around the base of a tooth or around the teeth. Indications of gingivitis can include swollen gums/gingiva, red gums/gingiva, bleeding gums/gingiva, receding gums/gingiva, tooth loss, and/or halitosis.

### SUMMARY

Technologies are disclosed for one or more scanner systems/devices, and/or techniques/methods that may be performed thereby, where the systems/devices may be configured for detection and/or display of an indication of a gingivitis condition of a subject's oral cavity via a digital representation of the oral cavity. The systems/devices may comprise a memory, a display device, and/or a processor. The processor may be configured to receive scan data of the oral cavity. The processor may be configured to indicate one or more teeth in the oral cavity based, at least in part, on the scan data. The processor may be configured to indicate a first assessment location proximate to a first tooth of the one or more teeth, based at least in part, on the scan data. The processor may be configured to generate a first image from the scan data. The first image may include at least the first assessment location and/or one or more first data channels. The one or more first data channels may comprise color data and/or topological information corresponding to the first assessment location.

The processor may be configured to determine, perhaps via one or more machine-learning algorithms, a first modified gingival index (MGI) value, a gingival index (GI), and/or another index related to a gingival condition, for the first assessment location based, at least in part, on the first image and/or the one or more first data channels. The processor may be configured to provide an indication of the first MGI value (e.g., a first modified gingival index (MGI) value, a first gingival index (GI) value, and/or another first index value related to a gingival condition) in a visually interpretable format via the digital representation of at least a part of the oral cavity on the display device.

Technologies are disclosed for one or more scanner systems/devices, or techniques/methods that may be performed thereby, where the systems/devices may be configured for detection and/or display of an indication of a gingivitis condition of a subject's oral cavity via a digital representation of the oral cavity. The systems/devices may comprise a memory, a display device, and/or a processor. The processor may be configured to receive scan data of the subject's oral cavity. The scan data may comprise a plurality of scans of the oral cavity that may be obtained from the subject over a period of time. The processor may be configured to indicate one or more teeth in the oral cavity based, at least in part, on the scan data. The processor may be configured to indicate a first assessment location proximate to a first tooth of the one or more teeth, based at least in part, on the scan data.

The processor may be configured to generate at least a first image from the scan data corresponding to a first time index. The first image may include at least the first assessment location at the first time index and/or a first set of one or more first data channels. The first set of one or more first data channels may comprise color data and/or topological information corresponding to the first assessment location at the first time index.

The processor may be configured to generate at least a second image from the scan data corresponding to a second time index. The second image may include at least the first assessment location at the second time index and/or a second set of one or more first data channels. The second set of one or more first data channels may comprise color data and/or topological information corresponding to the first assessment location at the second time index.

The processor may be configured to associate the first image with the second image as a pair. The processor may be configured to determine, perhaps via one or more machine-learning algorithms, a first differential modified gingival index (MGI) value (e.g., a first differential modified gingival index (MGI) value, a first differential gingival index (GI) value, and/or another first differential index value related to a gingival condition) for first assessment location, based, at least in part, on the first image, the second image, the first set of one or more first data channels, and/or the second set of one or more first data channels.

The processor may be configured to provide an indication of the first differential MGI value (e.g., a first differential modified gingival index (MGI) value, a first differential gingival index (GI) value, and/or another first differential index value related to a gingival condition) for the at first assessment location in a visually interpretable format via the digital representation of at least a part of the oral cavity on the display device.

Technologies are disclosed for one or more scanner systems/devices, and/or techniques/methods that may be performed thereby, where the systems/devices may be configured for detection and/or display of an indication of a periodontal pocket depth of a subject's oral cavity via a digital representation of the oral cavity. The systems/devices may comprise a memory, a display device, and/or a processor. The processor may be configured to receive scan data of the oral cavity. The processor may be configured to indicate one or more teeth in the oral cavity based, at least in part, on the scan data. The processor may be configured to indicate a first assessment location proximate to a first tooth of the one or more teeth based, at least in part, on the scan data.

The processor may be configured to generate a first image from the scan data. The first image may include at least the first assessment location and/or one or more first data channels. The one or more first data channels may comprise color data and/or topological information corresponding to the first assessment location.

The processor may be configured to determine, perhaps via one or more machine-learning algorithms, a first periodontal pocket depth value, and/or a first periodontal pocket depth value range, for the first assessment location based, at least in part, on the first image and/or the associated one or more first data channels. The processor may be configured to provide an indication of the first periodontal pocket depth value, and/or the first periodontal pocket depth value range for the first assessment location in a visually interpretable format via the digital representation of the oral cavity on the display device.

Technologies are disclosed for scanner systems/devices, and/or techniques/methods that may be performed thereby, where the systems/devices may be configured for detection and/or display of an indication of a periodontal pocket depth of a subject's oral cavity via a digital representation of the oral cavity. The systems/devices may comprise a memory, a display device, and/or a processor. The processor may be configured to receive scan data of the oral cavity. The scan data may comprise a plurality of scans of the oral cavity obtained from the subject over a period of time. The processor may be configured to indicate one or more teeth in the oral cavity based, at least in part, on the scan data. The processor may be configured to indicate a first assessment location proximate to a first tooth of the one or more teeth, based at least in part, on the scan data.

The processor may be configured to generate at least a first image from the scan data corresponding to a first time index. The first image may include at least the first assessment location at the first time index and/or a first set of one or more first data channels. The first set of one or more first data channels may comprise color data and/or topological information corresponding to the first assessment location at the first time index.

The processor may be configured to generate at least a second image from the scan data corresponding to a second time index. The second image may include at least the second assessment location at the second time index and/or a second set of one or more first data channels. The second set of one or more first data channels may comprise color data and/or topological information corresponding to the first assessment location at the second time index. The processor may be configured to associate the first image with the second image as a pair.

The processor may be configured to determine, perhaps via one or more machine-learning algorithms, a first differential periodontal pocket depth value for the first assessment location based, at least in part, on the first image, the second image, the first set of one or more first data channels, and/or the second set of one or more first data channels.

The processor may be configured to provide an indication of the first differential periodontal pocket depth value for the first assessment location in a visually interpretable format via the digital representation of at least a part of the oral cavity on the display device.

Technologies are disclosed for one or more techniques/methods, and/or processor-based devices/systems that may implement such techniques/device, where the techniques/methods may be for generating training data for systems/devices that may be configured to detect and/or display an indication of a gingivitis condition of a diagnostic subject's oral cavity via a digital representation of the diagnostic subject's oral cavity. The techniques/methods may comprise receiving scan data of a test subject's oral cavity. The techniques/methods may comprise indicating one or more teeth in the test subject's oral cavity based, at least in part, on the scan data. The techniques/methods may comprise indicating a plurality of assessment locations, based at least in part, on the scan data. One or more, or each of the plurality of assessment locations may be proximate to a tooth of the one or more teeth.

The techniques/methods may comprise generating a plurality of calibration images from the scan data. One or more, or each of the plurality of calibration images may include at least one assessment location of the plurality of assessment locations and/or at least one set of one or more data channels. One or more, or each of the at least one set of one or more data channels may comprise color data and/or topological information associated with the at least one assessment location.

The techniques/methods may comprise receiving a respective predetermined modified gingival index (MGI) (e.g., a predetermined modified gingival index (MGI) value, a predetermined first gingival index (GI) value, and/or another predetermined index value related to a gingival condition) for each of the plurality of calibration images. The techniques/methods may comprise associating each of the respective predetermined MGI with each of the calibration images and/or each of the at least one set of one or more data channels to form an MGI calibration data set (e.g., a modified gingival index (MGI) calibration data set, a predetermined first gingival index (GI) calibration data set, and/or another predetermined index calibration data set related to a gingival condition) for one or more machine-learning algorithms. The techniques/methods may comprise storing the MGI calibration data set (e.g., a modified gingival index (MGI) calibration data set, a predetermined first gingival index (GI) calibration data set, and/or another predetermined index calibration data set related to a gingival condition) into a memory of the computer processing device.

Technologies are disclosed for one or more techniques/methods, and/or processor-based devices/systems that may implement such techniques/device, where the techniques/methods may be for generating training data for systems/devices that may be configured to detect and/or display an indication of a periodontal pocket depth value of a diagnostic subject's oral cavity via a digital representation of the diagnostic subject's oral cavity. The techniques/methods may comprise receiving scan data of a test subject's oral cavity. The techniques/methods may comprise indicating one or more teeth in the test subject's oral cavity based, at least in part, on the scan data. The techniques/methods may comprise indicating a plurality of assessment locations, based at least in part, on the scan data. One or more, or each of the plurality of assessment locations may be proximate to a tooth of the one or more teeth.

The techniques/methods may comprise generating a plurality of calibration images from the scan data. One or more, or each of the plurality of calibration images may include at least one assessment location of the plurality of assessment locations and/or at least one set of one or more data channels. One or more, or each of the at least one set of one or more data channels may comprise color data and/or topological information associated with the at least one assessment location.

The techniques/methods may comprise receiving a respective predetermined periodontal pocket depth value, and/or a respective predetermined periodontal pocket depth value range, for one or more, or each of the plurality of calibration images.

The techniques/methods may comprise associating each of the respective predetermined periodontal pocket depth value, and/or or each of the respective predetermined periodontal pocket depth value range, with each of the calibration images and/or each of the at least one set of one or more data channels to form a periodontal pocket depth calibration data set for one or more machine-learning algorithms. The techniques/methods may comprise storing the periodontal pocket depth calibration data set into a memory of the computer processing device.

Technologies are disclosed for one or more techniques/methods, and/or processor-based devices/systems that may implement such techniques/device, where the techniques/methods may be for generating diagnostic data for systems/devices that may be configured to detect and/or display an indication of a gingivitis condition of a subject's oral cavity via a digital representation of the subject's oral cavity. The techniques/methods may comprise receiving scan data of the subject's oral cavity. The techniques/methods may comprise indicating one or more teeth in the subject's oral cavity based, at least in part, on the scan data. The techniques/methods may comprise indicating a plurality of assessment locations, based at least in part, on the scan data. One or more, or ach of the plurality of assessment locations may be proximate to a tooth of the one or more teeth.

The techniques/methods may comprise generating a plurality of diagnostic images from the scan data. One or more, or each of the plurality of diagnostic images may include at least one assessment location of the plurality of assessment locations and/or at least one set of one or more data channels. One or more, or each of the at least one set of one or more data channels comprising color data and/or topological information associated with the at least one assessment location.

The techniques/methods may comprise associating each of the diagnostic images and each of the at least one set of one or more data channels to form a MGI diagnostic data set (e.g., a modified gingival index (MGI) diagnostic data set, a gingival index (GI) diagnostic data set, and/or another index diagnostic data set related to a gingival condition) for one or more machine-learning algorithms. The techniques/methods may comprise storing the MGI diagnostic data set (e.g., a modified gingival index (MGI) diagnostic data set, a gingival index (GI) diagnostic data set, and/or another index diagnostic data set related to a gingival condition) into a memory of the computer processing device.

Technologies are disclosed for one or more techniques/methods, and/or processor-based devices/systems that may implement such techniques/device, where the techniques/methods may be for generating diagnostic data for systems/devices that may be configured to detect and/or display an indication of a periodontal pocket depth value of a subject's oral cavity via a digital representation of the subject's oral cavity. The techniques/methods may comprise receiving scan data of the subject's oral cavity. The techniques/methods may comprise indicating one or more teeth in the test subject's oral cavity based, at least in part, on the scan data. The techniques/methods may comprise indicating a plurality of assessment locations, based at least in part, on the scan data. One or more, or each of the plurality of assessment locations may be proximate to a tooth of the one or more teeth.

The techniques/methods may comprise generating a plurality of diagnostic images from the scan data. One or more, or each of the plurality of diagnostic images may include at least one assessment location of the plurality of assessment locations and/or at least one set of one or more data channels. One or more, or each of the at least one set of one or more data channels may comprise color data and/or topological information associated with the at least one assessment location included in the diagnostic image.

The techniques/methods may comprise associating each of the diagnostic images and each of the at least one set of one or more data channels to form a periodontal pocket depth diagnostic data set for one or more machine-learning algorithms. The techniques/methods may comprise storing the periodontal pocket depth diagnostic data set into a memory of the computer processing device.

Technologies are disclosed for one or more non-transitory computer readable mediums having instructions stored thereon. The instructions may cause at least one processor of an imaging analysis system/device to perform one or more operations. The imaging analysis system/device may be in communication with a display device. The one or more operations may comprise receiving scan data of a subject's oral cavity. The one or more operations may comprise indicating one or more teeth in the oral cavity based, at least in part, on the scan data. The one or more operations may comprise indicating a first assessment location proximate to a first tooth of the one or more teeth, based at least in part, on the scan data.

The one or more operations may comprise generating a first image from the scan data. The first image may include at least the first assessment location and/or one or more first data channels. The one or more first data channels may comprise color data and/or topological information corresponding to the first assessment location.

The one or more operations may comprise determining, perhaps via one or more machine-learning algorithms, a first modified gingival index (MGI) value (e.g., a first modified gingival index (MGI) value, a first gingival index (GI) value, and/or another first index value related to a gingival condition) for the first assessment location based, at least in part, on the first image and the one or more first data channels. The one or more operations may comprise providing an indication of the first MGI value in a visually interpretable format via a digital representation of at least a part of the oral cavity on the display device.

Technologies are disclosed for one or more non-transitory computer readable mediums having instructions stored thereon. The instructions may cause at least one processor of an imaging analysis system/device to perform one or more operations. The imaging analysis system/device may be in communication with a display device. The one or more operations may comprise receiving scan data of a subject's oral cavity. The scan data may comprise a plurality of scans of the oral cavity obtained from the subject over a period of time. The one or more operations may comprise indicating one or more teeth in the oral cavity based, at least in part, on the scan data. The one or more operations may comprise indicating a first assessment location proximate to a first tooth of the one or more teeth, based at least in part, on the scan data.

The one or more operations may comprise generating at least a first image from the scan data corresponding to a first time index. The first image may include at least the first assessment location at the first time index and/or a first set of one or more first data channels. The first set of one or more first data channels may comprise color data and/or topological information corresponding to the first assessment location at the first time index.

The one or more operations may comprise generating at least a second image from the scan data corresponding to a second time index. The second image may include at least the second assessment location at the second time index and/or a second set of one or more first data channels. The second set of one or more first data channels may comprise color data and/or topological information corresponding to the first assessment location at the second time index. The one or more operations may comprise associating a first image with a second image as a pair.

The one or more operations may comprise determining, perhaps via one or more machine-learning algorithms, a first differential modified gingival index (MGI) value (e.g., a first differential MGI value, a first differential gingival index (GI) value, and/or another first differential index value related to a gingival condition) for first assessment location, based, at least in part, on the first image, the second image, the first set of one or more first data channels, and/or the second set of one or more first data channels. The one or more operations may comprise providing an indication of the first differential MGI value (e.g., a first differential MGI value, a first differential gingival index (GI) value, and/or another first differential index value related to a gingival condition) for the at first assessment location in a visually interpretable format via a digital representation of at least a part of the oral cavity on the display device.

Technologies are disclosed for one or more non-transitory computer readable mediums having instructions stored thereon. The instructions may cause at least one processor of an imaging analysis system/device to perform one or more operations. The imaging analysis system/device may be in communication with a display device. The one or more operations may comprise receiving scan data of a subject's oral cavity. The one or more operations may comprise indicating one or more teeth in the oral cavity based, at least in part, on the scan data. The one or more operations may comprise indicating a first assessment location proximate to a first tooth of the one or more teeth based, at least in part, on the scan data.

The one or more operations may comprise generating a first image from the scan data. The first image may include at least the first assessment location and/or one or more first data channels. The one or more first data channels may comprise color data and/or topological information corresponding to the first assessment location.

The one or more operations may comprise determining, perhaps via one or more machine-learning algorithms, a first periodontal pocket depth value, and/or a first periodontal pocket depth value range, for the first assessment location based, at least in part, on the first image and the one or more first data channels. The one or more operations may comprise providing an indication of the first periodontal pocket depth value, and/or the first periodontal pocket depth value range for the first assessment location in a visually interpretable format via a digital representation of the oral cavity on the display device.

Technologies are disclosed for one or more non-transitory computer readable mediums having instructions stored thereon. The instructions may cause at least one processor of an imaging analysis system/device to perform one or more operations. The imaging analysis system/device may be in communication with a display device. The one or more operations may comprise receiving scan data of a subject's oral cavity. The scan data may comprise a plurality of scans of the oral cavity obtained from the subject over a period of time. The one or more operations may comprise indicating one or more teeth in the oral cavity based, at least in part, on the scan data. The one or more operations may comprise indicating a first assessment location proximate to a first tooth of the one or more teeth, based at least in part, on the scan data.

The one or more operations may comprise generating at least a first image from the scan data corresponding to a first time index. The first image may include at least the first assessment location at the first time index and/or a first set of one or more first data channels. The first set of one or more first data channels may comprise color data and/or topological information corresponding to the first assessment location at the first time index.

The one or more operations may comprise generating at least a second image from the scan data corresponding to a second time index. The second image may include at least the second assessment location at the second time index and/or a second set of one or more first data channels. The second set of one or more first data channels may comprise color data and/or topological information corresponding to the first assessment location at the second time index. The one or more operations may comprise associating a first image with a second image as a pair.

The one or more operations may comprise determining, perhaps via one or more machine-learning algorithms, a first differential periodontal pocket depth value for the first assessment location based, at least in part, on the first image, the second image, the first set of one or more first data channels, and/or the second set of one or more first data channels. The one or more operations may comprise providing an indication of the first differential periodontal pocket depth value for the first assessment location in a visually interpretable format via a digital representation of at least a part of the oral cavity on the display device.

### BRIEF DESCRIPTION OF DRAWINGS

The elements and other features, advantages and disclosures contained herein, and the manner of attaining them, will become apparent and the present disclosure will be better understood by reference to the following description of various examples of the present disclosure taken in conjunction with the accompanying drawings, wherein:
FIG. 1 is an example illustration of a tooth with a healthy section and a diseased section;
FIG. 2 is an example illustration of a healthy tooth, a tooth with gingivitis, and a tooth with periodontitis;
FIG. 3A and FIG. 3B illustrate an example of gingival tissues surrounding a given tooth that have been segmented into several sites/assessment locations for analysis;
FIG. 4 is a block diagram of a hardware configuration of an example device that may control one or more parts of a machine-learning algorithm analysis of intra-oral 3D scan data;
FIG. 5 is an example of 2D image color data and topological information exported from 3D scan data for one or more virtual probing sites/assessment locations;
FIG. 6 illustrates an example of multiple virtual probing sites/assessment locations corresponding to the gingiva tissue proximate to three teeth of a subject's oral cavity;
FIG. 7 illustrates an example output from an IOS 3D scan of a subject's oral cavity;
FIG. 8A and FIG. 8B illustrate an example of a tooth identification, gingival tissue identification, and an assessment location/virtual probing site determination;
FIG. 9 illustrates a visual example of color data and/or topological information represented by one or more data channels of one or more images/snapshots;
FIG. 10A and FIG. 10B is an example illustration of a segmentation of a tooth of a set of teeth;
FIG. 11A, FIG. 11B, FIG. 11C, and FIG. 11D is an example illustration of an alignment of a tooth between two scans made over a period of time;
FIG. 12 is a flowchart of an example technique for a cross-sectional assessment of a gingivitis condition of a subject's oral cavity;
FIG. 13A and FIG. 13B is a flowchart of an example technique for a longitudinal assessment of a gingivitis condition of a subject's oral cavity;
FIG. 14 is a flowchart of an example technique for a cross-sectional assessment of a periodontal pocket depth of a subject's oral cavity; and
FIG. 15A and FIG. 15B is a flowchart of an example technique for a longitudinal assessment of a periodontal pocket depth of a subject's oral cavity.

### DETAILED DESCRIPTION

For the purposes of promoting an understanding of the principles of the present disclosure, reference will now be made to the examples illustrated in the drawings, and specific language will be used to describe the same. It will nevertheless be understood that no limitation of the scope of this disclosure is thereby intended.

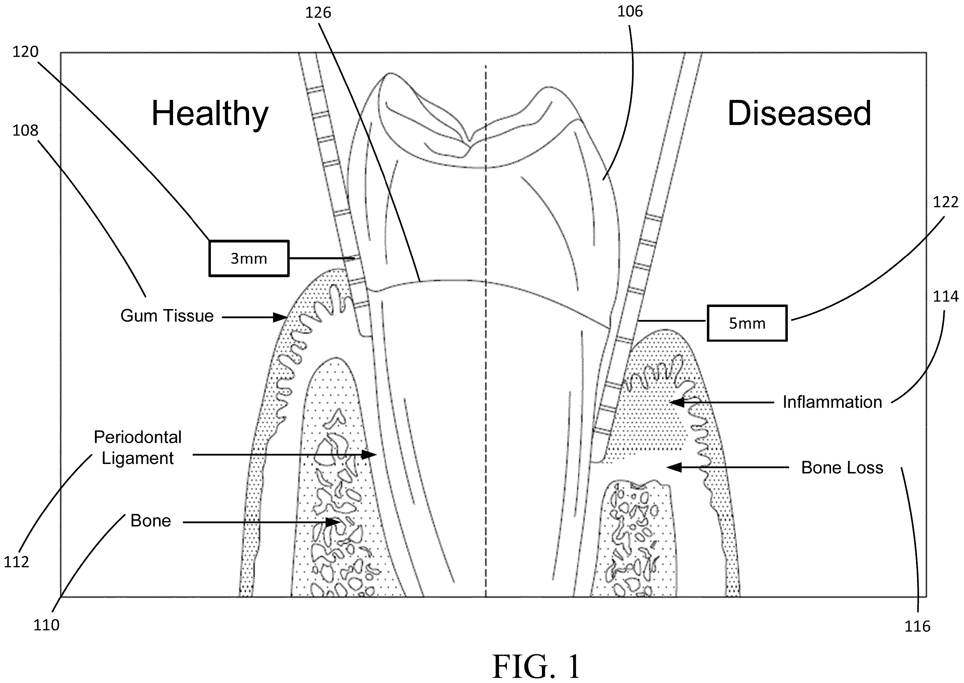

FIG. 1 illustrates an example cross-section model of a tooth 106 rooted into a gum/gingiva tissue/substrate 108 that may have a "healthy" section and a "diseased" section. Bone structure 110 are present around the tooth 106, as is periodontal ligament(s) 112. Inflammation 114 may be indicative of dental disease, as could bone loss 116 on the right side of the tooth 106. A pocket 120 with a depth of 3 mm or less may be indicative of a healthy gum/gingiva tissue 108. A pocket 122 with a depth of greater than 3 mm (e.g., 5mm) may be indicative of dental disease. A cementoenamel junction (CEJ) 126 is illustrated on the tooth 106.

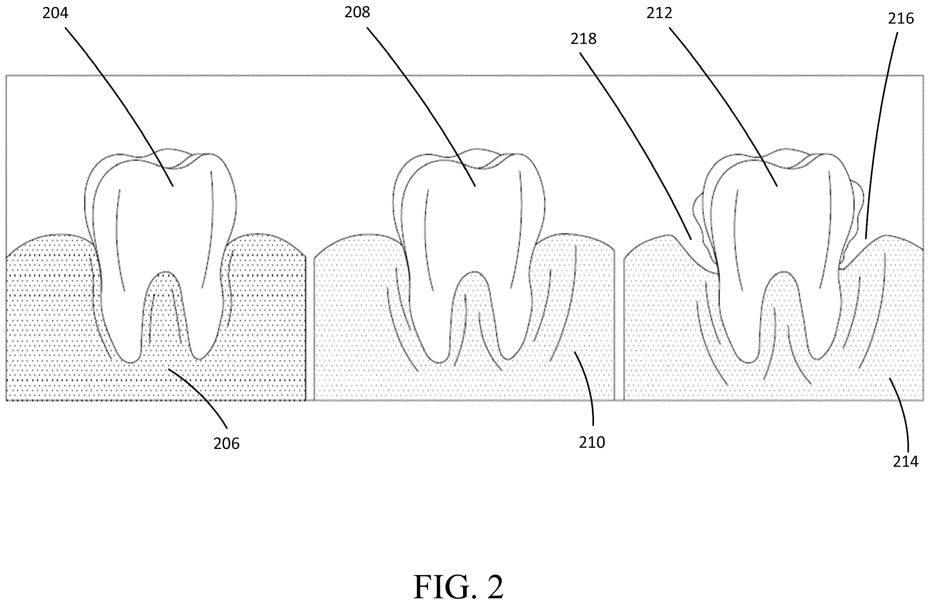

FIG. 2 illustrates relatively general examples of three different conditions of a tooth/gum pairing. A tooth 204 may be rooted in a generally healthy gum 206. A tooth 208 may be rooted in a gum 210 that may demonstrate at least some degree of gingivitis. A tooth 212 may be rooted in a gum 214 that may or might not demonstrate at least some degree of gingivitis. The gum 214 may include periodontal pockets 216 and/or 218 that may indicate at least some degree of periodontitis.

Gingivitis is an inflammatory condition of the gingival tissue, most often caused by bacterial infection. Gingivitis is characterized by swelling, redness, exudate, a change of normal contours, bleeding, and, occasionally, discomfort in the gum/gingiva area of the oral cavity. Gingivitis affects over 90% of the world population to some degree and is prevalent at all ages.

The manifestations of gingival inflammation are vascular changes including increased volume of crevicular fluid and increased blood flow at the marginal gingival region. Clinically, gingiva may appear with edema, less stippled, and more red than healthy gingiva. Diagnosis of gingivitis by the clinician relies on the identification of signs and symptoms of inflammation resulting from the disease process in the gingival tissues, and may be based on inspection of the color, texture, edema of gingiva, and bleeding on probing. The assessment may be noninvasive using a visual technique, and/or may be invasive using instrumentation. Bleeding is an early sign of gingivitis and clinical assessment is often based on invasive use of a periodontal probe, and the suitability of bleeding provocation in the gingival margin is dependent on the probing pressure. Often, measurement of the gingival inflammation is subjective in nature and requires expert examiner training and knowledge to examine a patient at multiple sites to arrive at an appropriate diagnosis and/or management strategy. The assessment can be time-consuming and/or uncomfortable for the patient.

Gingivitis is reversible with professional treatment, patient motivation, and good oral hygiene instruction, which is useful for regenerating healthy gingiva without irreversible damage. Patients are often unaware that they are suffering from gingivitis before it is diagnosed and being demonstrated/indicated to them when they attend a dental appointment. Gingivitis is the first and mildest stage of progression of periodontal disease. If left untreated, gingivitis can lead to the presence of an abnormal depth of the gingival sulcus (periodontal pocket), loss of jawbone surrounding the teeth, and eventually tooth loss. Therefore, an early diagnosis of gingivitis is crucial for preventing periodontal diseases.

Development of non-invasive techniques which could predict the changes in microcirculation and morphology related to dental diseases like gingivitis would be very useful in the diagnosis and/or monitoring of gingival/periodontal diseases. Digital cameras with color calibration/software show that changes in gingival health assessed by standard non-invasive gingival indices may also be detected as a change in gingival redness on a digital image. Digital imaging may be used to record gingival inflammation by changes in redness.

Periodontal disease (e.g., periodontitis) is a set of inflammatory conditions affecting the tissues surrounding the teeth, starting with gingivitis in its early stage. In the more serious form of periodontitis, gingiva can pull away from the tooth and a space between the tooth and the surrounding gingiva is formed (e.g., a periodontal pocket). Periodontal pockets provide an ideal environment for bacteria to grow and/or may spread infection to the structures that keep teeth anchored in the mouth, and the underlying bone may likely be destroyed (e.g., bone loss). As periodontal disease advances leading to more bone loss, the teeth may loosen or fall out.

The prevalence of periodontitis is high and 47.2% of adults aged 30 years and older in the U.S. have some form of periodontal disease. The prevalence increases with age where 70.1% of adults 65 years and older have periodontal disease.

The most frequently used diagnostic tool for assessing the health status and attachment level of the tissues surrounding the teeth is a periodontal probe, which is placed between the gingiva and the tooth. This clinical assessment is very time-consuming and thus also expensive, is unpleasant for the patient, and also lacks reproducibility in and among examiners. For a reasonably detailed periodontal chart, typically, at least six (6) sites per tooth are probed. For full-mouth pocket depth measurement, the examiner (e.g., dentist or the hygienist) may use up to twenty (20) minutes for probing and measuring the pocket depth of a patient/subject. Traditional periodontal probing may suffer from insufficient reproducibility and accuracy, due to significant differences in individual techniques, probe instruments, and variation in probing depth force, even among different operators/examiners or even the same operator/examiner at different times. The resulting errors can impact clinical decision-making, especially during longitudinal (e.g., over a period of time) monitoring of the periodontal status.

A clinically applicable, non-invasive and reproducible method to detect the presence of gingivitis and/or estimate the periodontal pocket depth and/or monitor these diseases with high precision may be very useful, at least to address one or more of the issues related to traditional dental practices described herein.

Intra oral scanners (IOS) can be highly accurate. IOS techniques and IOS digital data analysis are more accurate and reproducible than using a periodontal probe to measure the width of keratinised gingiva, compared to conventional probe data. Three-dimensional (3D) IOS images of gingiva have great potential and use for efficient and accurate data capture for clinical evaluation of health and/or inflammatory gingival status and measuring pocket depth using non-invasive IOS techniques and IOS digital data analysis.

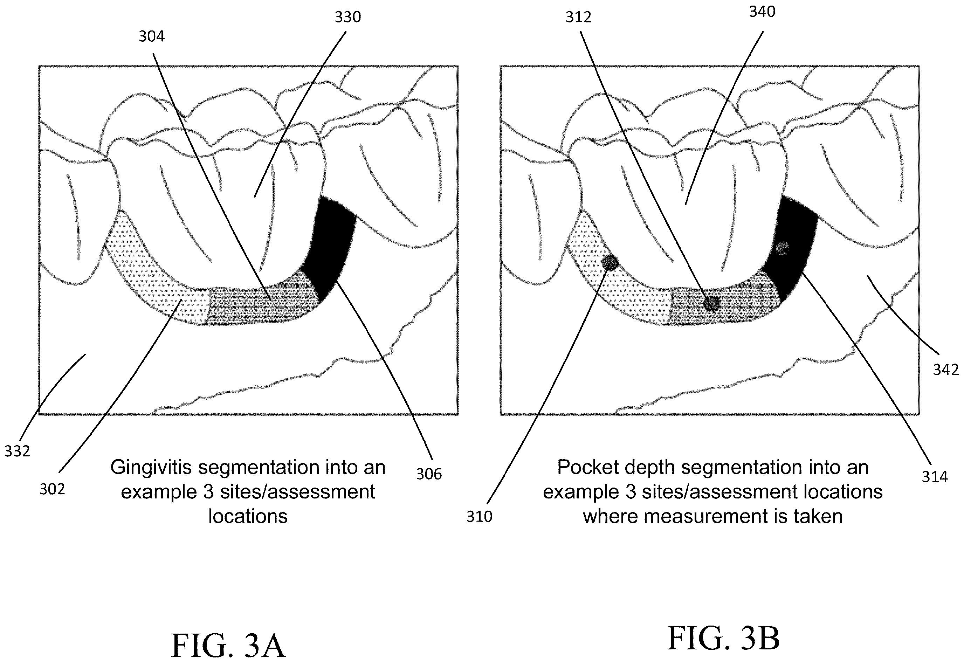

Technologies for deep learning/machine-learning algorithms for pocket depth assessment and gingivitis detection are described herein. These algorithms may process full 3D scan models and return estimates of periodontal pocket depth and/or severity of gingivitis for the relevant sites on the gingiva. FIG. 3A and FIG. 3B illustrate an example of gingival tissues surrounding a given tooth that have been segmented into three (3) sites/assessment locations. The three sites/assessment locations are for example, and more sites/assessment locations or less sites/assessment locations are contemplated and derivable from the technologies and/or techniques described herein.

In FIG. 3A, the gingival tissues surrounding a given tooth 330 rooted in the gum 332 can be segmented into 3 sites: mesial (302), middle (304) and distal (306). This gingivitis/periodontal pocket segmentation is an example of 3 sites/assessment locations, as more or less sites/assessment locations are derivable. The machine learning algorithms may return estimates of periodontal pocket depth and/or MGI (e.g., a MGI value, a gingival index (GI) value, and/or another index value related to a gingival condition) scores for one or more, or each, of those sites/assessment locations. In FIG. 3B, for a tooth 340 rooted in the gum 342 pocket depth segmentation can be performed into an example three (3) sites/assessment locations 310, 312, and 314, where pocket depth measurements may be taken, for example.

In one or more scenarios, there may be at least two types of assessments made for gingivitis and/or periodontal pocket depth/pocket depth - cross-sectional and/or longitudinal. For example, cross-sectional assessment may include the detection and/or diagnosis of disease at one particularly point in time. For example, longitudinal assessment may include changes in disease severity over some period of time. Cross-sectional and longitudinal algorithms for periodontal pocket depth assessment and gingivitis detection are described herein.

One or more machine-learning (ML) algorithms are contemplated to be very effective in the analysis of IOS digital data, as the increase of pocket depth over time results in morphological changes on the corresponding soft dental tissues. And for gingivitis, varying degrees of inflammation lead to both morphological changes and changes in the visual appearance of the gingival tissues. A neural network using one or more ML algorithms trained with/on a sufficient amount of high-quality data may recognize and/or assess these morphological changes and/or visual characteristics.

In one or more scenarios, pocket depth and/or gingivitis data may be collected from clinical examination (e.g., IOS digital data, physical examination data, etc.). Examiners can get very different results, when scoring pocket depth and/or when assessing gingivitis. Thus, data from multiple examiners may be useful for training and/or diagnostic analysis. Such data may be used as standard reference and train neural networks on intra-oral scan data analysis. The accuracy of the time-consuming pocket depth measurement is not high. The well-selected training data may compensate for the scanner in that it might not measure the pocket depth directly, but could assess pocket depth from the morphology of the soft tissue. The results from the intra-oral scan (IOS) are expected to be much more reproducible which is an advantage for comparing over time (e.g., monitoring).

Pocket depth measurement and/or gingivitis assessment may be time-consuming, expensive, and lacks reproducibility. The replacement of the existing methods by an intra-oral scan may save time and/or may allow analysis of these parameters at more visits and for more patients, perhaps for example because it will not require much extra time, and may potentially spot changes and/or periodontal disease earlier. Often, pocket depth measurement might be performed on a few patients and perhaps if (e.g., only if) a severe risk for this condition is found. Assessment of these conditions from intra-oral scan digital data may allow for screening of more, maybe almost all patients, and these conditions might be detected earlier when prevention still has higher chances of success and/or severe deep pockets can be prevented. An intra-oral scan may take only 3-10 minutes and/or may pose no harm (e.g., due to ionizing radiation, etc.). Such analysis of IOS digital data my offer the ability of frequent analysis for more patients and/or an earlier warning that may trigger pocket depth measurement assessment, perhaps in the classical way, among other ways.

In one or more scenarios, cross-sectional ML algorithm analysis (e.g., on a one/single scan), and/or longitudinal ML algorithm (e.g., on two or more scans, etc.) may be performed. Cross-sectionally, ML algorithm analysis may predict/estimate the pocket depth and/or gingivitis from the outer shape of the soft tissue, perhaps together with the gingival color, the shape and position of the gingival margin (e.g., in relation to the CEJ-cement enamel junction, among other topological information). Longitudinally, ML algorithm analysis of IOS digital data (e.g., 3D scans, etc.) may be reliable considering volumetric changes, shape changes, surface shape changes, and/or the color of the soft tissue changes between the IOS scans from different times, among other reasons.

In one or more scenarios, for example for training, among other scenarios, intra-oral three-dimensional (3D) scans of many patients (e.g., 10-1000, among other ranges) may be collected, perhaps together with pocket depth measurement results of six (6) sites/assessment locations per tooth of one or more teeth in a subject's oral cavity. In one or more scenarios, data from more than six (6) sites/assessment locations could be used, or data from less than six (6) sites/assessment locations could be used. Machine learning-based segmentation algorithms/networks may segment the intra-oral scan data into one or more subparts, for example teeth and soft tissues. One or more ML algorithms may (e.g., automatically) identify six (6) sites per tooth which may correspond to the sites/assessment locations where pocket depth measurements are usually performed.

The segmentation algorithm(s) may identify buccal/facial and/or lingual/palatal direction of the teeth. For one or more, or each site/assessment location, one or more, or multiple (e.g., 1-100, etc.) images (e.g., "artificial" 2D snapshots, etc.) of the IOS 3D digital data may be created from one or more different viewing angles and/or with varying magnification and/or resolution. Snapshots similar to color images (e.g., red, green, and/or blue, etc.) and/or snapshots representing/including the topology of the 3D surface may be generated. One or more of the snapshots may be captured by a "virtual camera" that may process the 3D scan data into the snapshots and/or one or more images (e.g., 2D images) that may be associated with topological data from the 3D scan data.

In one or more scenarios, the use of a 2D snapshot/images obtained from the 3D scan data may provide color data/information and/or topological data/information, such as depth data/information, curvature data/information, facet normal data/information, distance data/information (e.g., distance to the virtual camera, and/or other reference points).

In one or more scenarios, the color data/information may be useful for gingivitis and/or pocket depth detection, as the changes in color of the gingiva may indicate gingival conditions and/or periodontal pocket issues. The depth data/information may be a measure of the distance between the virtual camera, among other reference points, and the surfaces in the oral cavity. The curvature may provide a measure of the (e.g., rapid, subtle, etc.) changes that may occur on the surface of the model. This could be an indication of a number of issues such as for example swelling of the gingiva, among other issues/conditions. One or more facet normal may be used to generate image information that may contain information regarding the direction of shape of the data. This may be useful when especially detecting gingivitis (e.g., swelling etc.) and/or pocket depth conditions/issues.

As an in vivo pocket depth measurement may have been carried out at the time of the data collection, one or more, or each of these 2D snapshots/images may exported from the 3D model/digital scan data may have a corresponding pocket depth label. The data can be used for training the ML algorithms, validating the ML algorithm analysis, and/or testing the ML algorithms. The training/validating/testing data set(s) are contemplated to be large enough to allow for statistically significant ML algorithm analysis of the diagnostic IOS 3D digital data.

In one or more scenarios, for example after training of the ML algorithms for pocket depth measurement and/or gingivitis indication, one or more diagnostic techniques may include receiving IOS 3D scan data. The 3D scan data may be tooth, gum/gingiva, and one or more virtual probing sites/assessment locations. One or more images (e.g., 2D snapshots) that may contain color data and/or topology information of the 3D surface models may be generated and passed to the ML algorithm analysis. The one or more ML algorithms may return analysis estimates of pocket depth measurements for one or more of the sites/assessment locations.

The pocket depth measurements/estimates may be displayed via one or more display devices in communication with the ML algorithms. The measurements can be displayed as a number/value per site/assessment location on a visual rendering/depiction of 3D model. The measurements can be displayed as an average and/or median pocket depth per tooth, and/or per lingual/palatal and buccal/facial (e.g., 2 per tooth, etc.). The measurement data may be exported into a csv and/or excel file format, or similar format, so that, for example, the measurement data may be exported into an electronic dental chart or a format readable by third party dental chart software, or the like.

The gingivitis ML algorithm analysis may function very much like the pocket depth measurement ML algorithm analysis described above. For gingivitis assessment, the output may be an estimated modified gingival index (MGI) (e.g., a MGI value, a gingival index (GI) value, and/or another index value related to a gingival condition) score rather than, or in addition to, a pocket depth measurement. Training data may be obtained by collecting intra-oral 3D scans of a large number of patients and performing an MGI score (e.g., a MGI score, a gingival index (GI) score, and/or another index score related to a gingival condition) assessment on, for example, six sites per tooth (the virtual probing sites/assessment locations). In similar fashion to the pocket depth training data, each 2D snapshot of a virtual probing site/assessment location may have a corresponding MGI score (e.g., a MGI score, a gingival index (GI) score, and/or another index score related to a gingival condition) label, for example.

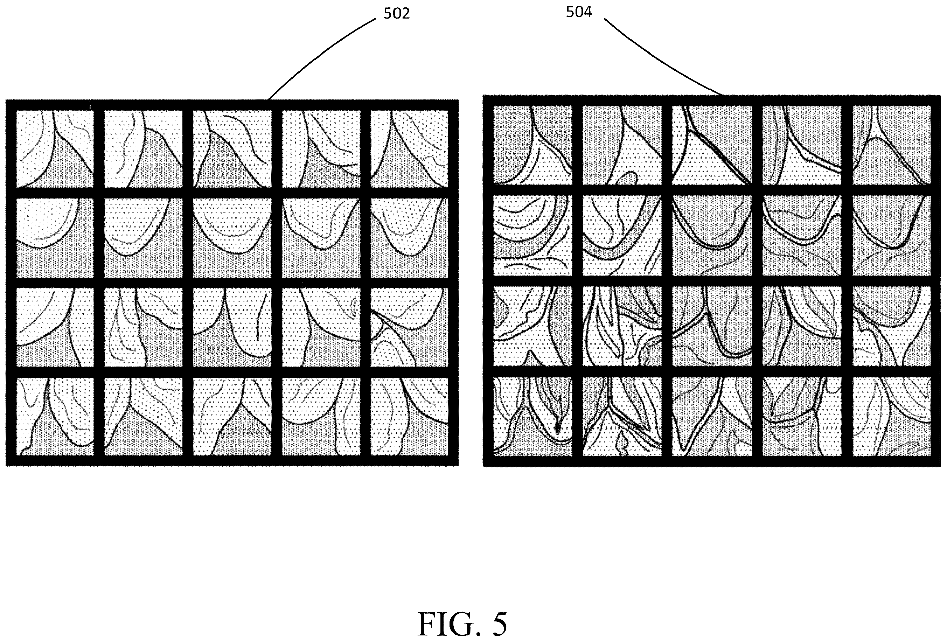

FIG. 5 illustrates an example of 2D red-green-blue (RGB) images/color data 502 (adapted for black & white representation) that may be exported from 3D scan data for the one or more virtual probing sites/assessment locations. At 504, an example of 2D topology information/images (e.g., facet normals) (adapted for black & white representation) may be exported from the 3D scan data for the one or more virtual probing sites/assessment locations. The data illustrated in FIG. 5 may be useful for gingivitis analysis and/or periodontal pocket depth analysis/detection.

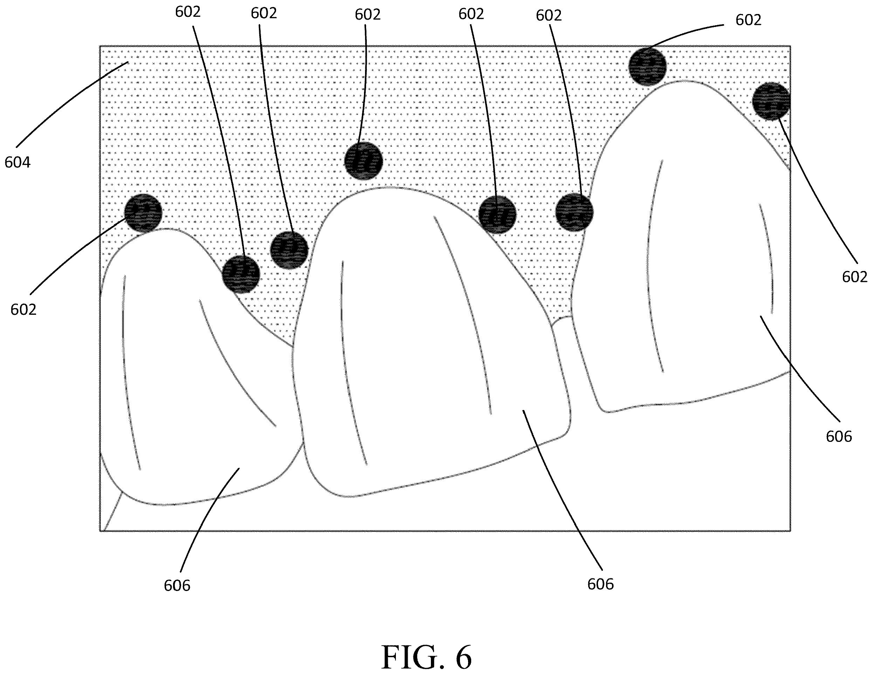

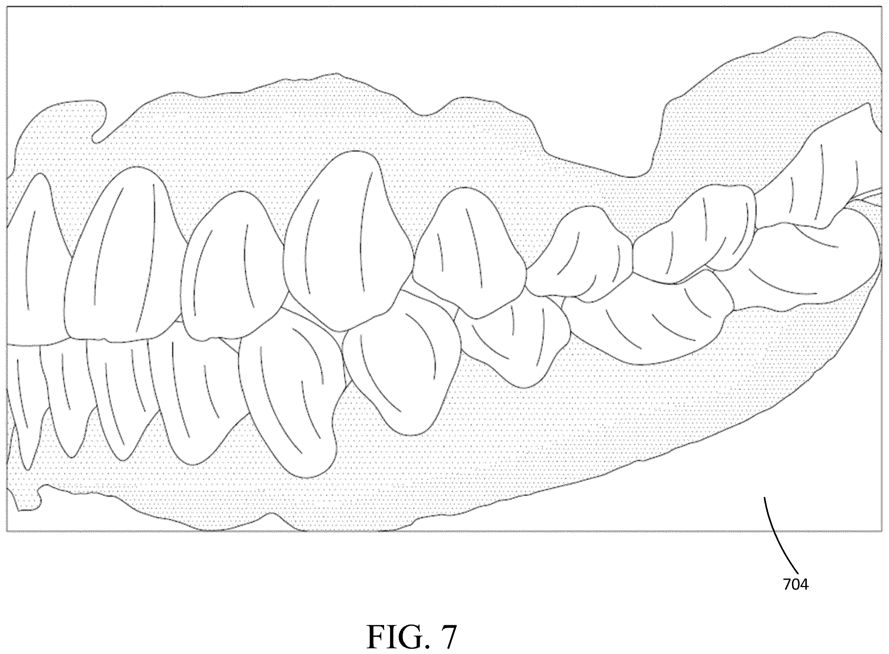

FIG. 6 illustrates an example of eight (8) virtual probing sites/assessment locations 602 corresponding to the gum/gingiva tissue 604 proximate to three teeth 606 of a subject's oral cavity. In one or more scenarios, the ML algorithm analysis may provide measurements/estimates for an MGI values (e.g., a MGI values, a gingival index (GI) values, and/or another index value related to a gingival condition) and/or pocket depth measurements for the one or more sites/assessment locations 602. FIG. 7 illustrates an example image/model 704 from an IOS 3D scan. The image/model 704 may illustrate a 3D model with 3D information (e.g., topology data/information).

In one or more scenarios, criteria for the Modified Gingival Index (MGI) may be expressed as a numerical scale that may include one or more of the value "0" as an absence of inflammation, the value "1" as a mild inflammation - slight change in color, little change in texture of any portion of but not the entire marginal or papillary gingival unit, the value "2" as a mild inflammation - that may include similar criteria as for value "1" but involving the entire marginal or papillary gingival unit, the value "3" as a moderate inflammation - glazing, redness, edema, and/or hypertrophy of the marginal or papillary gingival unit, and/or the value "4" as a severe inflammation - marked redness, edema and/or hypertrophy of the marginal or papillary gingival unit, spontaneous bleeding, congestion, and/or ulceration.

In one or more scenarios, a tooth may be identified/selected/determined and/or at least one side of the tooth may be identified/selected/determined (e.g., a buccal-lingual side of the tooth). The neighbouring gingiva facets within some distance of the tooth can be identified in/with a flood fill fashion, among other techniques. In one or more scenarios, the flood fill algorithm is run with perhaps two conditions/considerations, such as consider a "maximum" distance away from the identified tooth, and/or never choose gingiva facets corresponding to/belonging to other teeth.

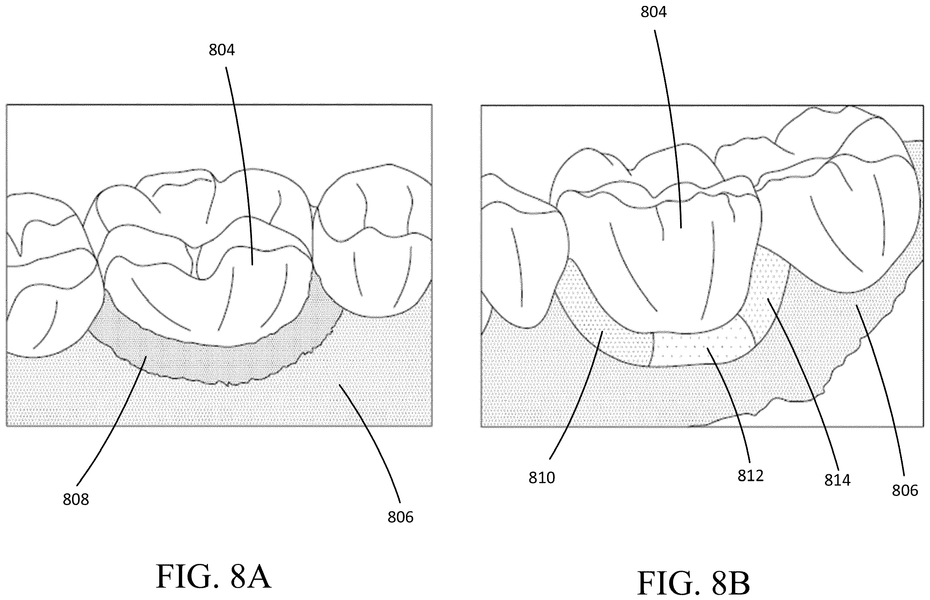

FIG. 8A and FIG. 8B illustrate an example of a tooth identification, gingival tissue identification, and an assessment location/virtual probing site determination. In FIG. 8A, one or more segmentation algorithms may identify a tooth 804 and/or may identify gingiva tissue 806 corresponding to the tooth 804. A 3 mm "band" 808 of the gingiva may be identified via flood filling neighbouring facets, for example. In one or more scenarios, other size bands may be identified. In FIG. 8B, the gingival band 808 can be subdivided into three (3) virtual probing sites/assessment locations 810, 812, and 814 that may correspond to clinical examination sites, for example. In one or more scenarios, less than three sites/assessment locations, or more than three sites/assessment locations may be identified.

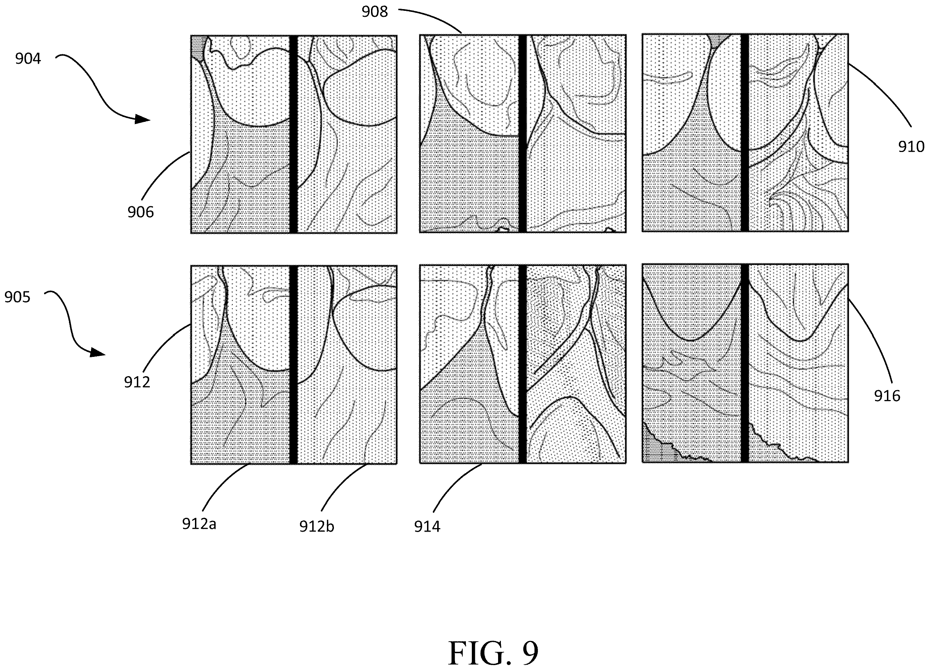

In one or more scenarios, 2D images/snapshots may be acquired with any type of virtual camera and/or using the center of mass and/or the average facet normal direction of the virtual probing site/assessment location to determine its position. The 2D images/snapshots can contain color data and/or topological information. For example, six (6) data channels may include red, green, and/or blue color data and/or facet normal Nx, Ny, Nz components (e.g., in camera coordinates). FIG. 9 illustrates visually what may be represented by one or more data channels, where for example there may be six (6) data channels per 2D image/snapshot and/or assessment location/site conveying color data and/or topological information (e.g., facet normals). FIG. 9 represents a sample from the data, showing 2D images/snapshots of the virtual probing sites/assessment locations as acquired from an example tooth. As an example, referring to FIG. 8B, tooth 804 may have three virtual probing sites/assessment locations 810, 812, 814 for which one or more 2D snapshots have been captured.

Examples of such 2D snapshots from one or more probing sites of a (e.g., single) tooth are reflected in FIG. 9. In FIG. 9, six (6) example snapshots 906, 908, 910, 912, 914, and 916 are illustrated. The top three (3) snapshots 906, 908, and/or 910, for example, may represent three (3) virtual probing sites/assessment locations from the buccal side of a tooth. The bottom three (3) snapshots 912, 914, and/or 916, for example may represent three (3) virtual probing sites/assessment locations from the lingual side of the same tooth. One or more, or each, of the illustrated six (6) snapshots 906, 908, 910, 912, 914, and/or 916 may comprise one or more data channels, for example six (6) data channels. For example, snapshot 912 could correspond to the virtual probing site/assessment location 810 of FIG. 8B. For example, snapshot 912 may comprise, and/or may be associated with, at least one color data channel 912a and/or at least one topological data channel 912b. The color data channel 912a may comprise at least one of a red, green and/or blue color information (adjusted for representation in black and white). The topological data channel 912b may comprise at least one of a facet normal information such as the Nx, Ny, and/or Nz components. One or more, or each, of these snapshots and the corresponding data channels may be input to the one or more neural networks/ML algorithms described herein to obtain (e.g., optimal) detection of pocket depth measurements and/or gingivitis assessments.

In one or more scenarios, one or more convolutional neural networks/models (CNNs) may be a part of one or more ML algorithms. The CNNs may be set up such that they are compatible with the 2D images/snapshots that may be generated from the virtual probing sites/assessment locations from the 3D digital data/model. In one or more scenarios, there may be six (6) probing sites with three (3) on each side of a tooth for one or more, or each tooth in a set of the teeth in a dentition. In one or more scenarios, there could be more than three sites/assessment locations per side of each tooth, or less than three sites/assessment locations per side of each tooth.

The one or more CNNs/models may be configured to perform a series of, for example, filtering operations on the 2D images/snapshots to achieve a compressed representation of the various input 2D images/snapshots. This stage of the CNN/model may be referred to as the encoder.

One or more other stages of the CNNs/models, for example the classification head may reason/process about the compressed input representation and assign to it a score or a label (for gingivitis and/or pocket depth measurement). For the cross-sectional assessments,_the CNNs/models produce scores according to the MGI scale (e.g., a MGI scale, a gingival index (GI) scale, and/or another index scale related to a gingival condition). During training, the CNNS/models and their parameters may be iteratively updated to minimize an objective function (e.g., a loss function) that penalizes the CNNs/models for making wrong predictions/inferences/assessments.

For training, as described herein, the correct MGI/pocket depth scores/values are known from clinical trials. Given a sample of training examples (e.g., 2D images/snapshots from the virtual probing sites/assessment locations), the objective function expressed as a function of the CNNs/models parameters may comprise a loss surface/landscape on which a search for a global minimum may be done using standard optimization methods.

For longitudinal assessments, the CNNs/models differ from the cross-sectional configuration in that instead of absolute MGI/pocket depth scores, the CNNs/models output MGI/pocket depth score differences between two 2D images/snapshots of the same virtual probing site/assessment location taken from two different times. Another difference may be that the two images may be passed through the same CNN/model encoder and their compressed representations may be concatenated and fed to a classification head.

The training examples used to train these longitudinal CNN/models as image pairs, where the two images in an image pair come from the same virtual probing site, but from different scans of the same patient/subject (e.g., from different scans taken at different points in time - that could be different times on the same day or different times with days, weeks, or months in between). The correct scores for both 2D images/snapshots are known from clinical trials and the CNN/model target becomes the difference between them (e.g., ordered in time as Score_scan1-Score_(scan2_ )).

As described herein, in one or more scenarios, segmentation may be conducted on the 3D digital scan data and/or the 2D images/snapshots. The segmentation may be conducted on one 3D digital scan data/representation, and/or on at least a first and a second 3D digital scan data representation. The segmentation may identify the parts of the one 3D digital can data/representation, and/or on the first and second 3D digital scan data/representations that may correspond to different teeth of a set of teeth/dentition. The segmentation may provide that the identified teeth and/or the gingiva tissue can be separated and/or treated as independent 3D models of the individual teeth and/or the gingiva, for example.

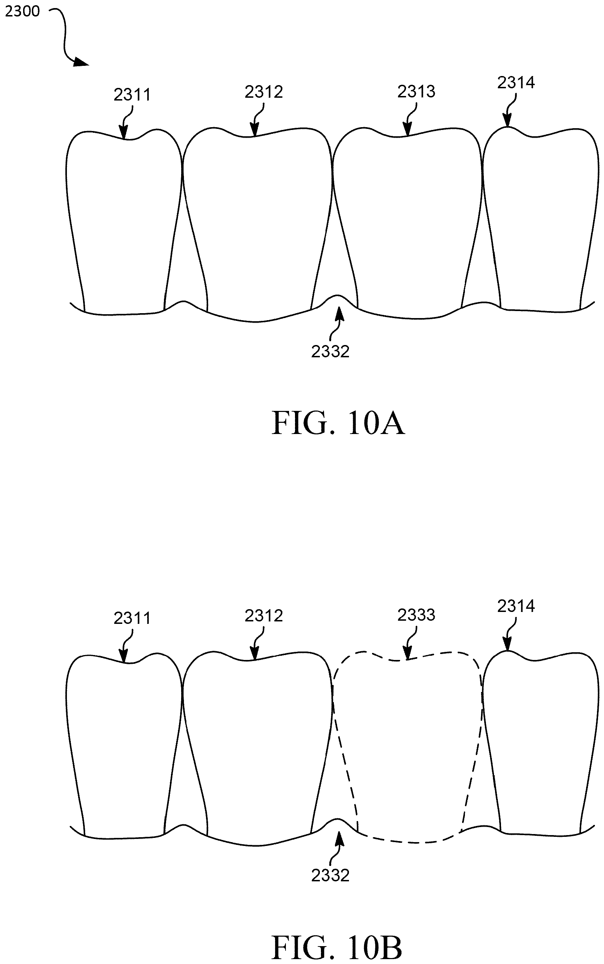

FIG. 10A shows an example 3D digital scan data/representation of a set of teeth and segmentation of the 3D digital scan data/representation to create a 3D model of a tooth. The 3D digital scan/representation 2300 has topography/topology information/data for four anterior teeth 2311, 2312, 2313, and 2314 and for a portion of the corresponding gingiva with the gingival boundary 2332 as indicated. The segmentation of the 3D digital scan data/representation provides a 3D tooth model 2333 as shown in FIG. 10B which has the shape of the corresponding tooth part of the 3D digital scan data/representation 2313 and is bounded by the gingival boundary 2332. In FIG. 10B, the 3D tooth model 2333 is still arranged along with the other parts of the 3D digital scan data/representation according to the arrangement of the teeth in the 3D digital scan/representation.

In one or more scenarios, a local alignment of segmented teeth of a first and second 3D digital scan/representations may be conducted. In the context of the present application, the phrase "locally aligning segmented teeth" refers to the situation where one or more of the segmented teeth of one 3D digital scan/representation may be individually aligned with the corresponding teeth of another 3D digital scan/representation. The local alignment may be realized by aligning corresponding digital 3D models of the teeth extracted from the 3D digital scan/representations, such as for example aligning digital 3D models of a canine or human tooth extracted from the 3D digital scan/representations. The 3D models of the segmented teeth of the first 3D digital scan/representation may be aligned with the corresponding 3D models of the teeth in the second 3D digital scan/representation on a tooth-to-tooth basis, for example. In one or more scenarios, the alignment may be local on the scale of the individual teeth rather than on a global scale of the entire set of teeth. Transformations used for aligning different segmented teeth may be different in contrast to a global alignment where the same transformation is applied to all the teeth and the gingiva.

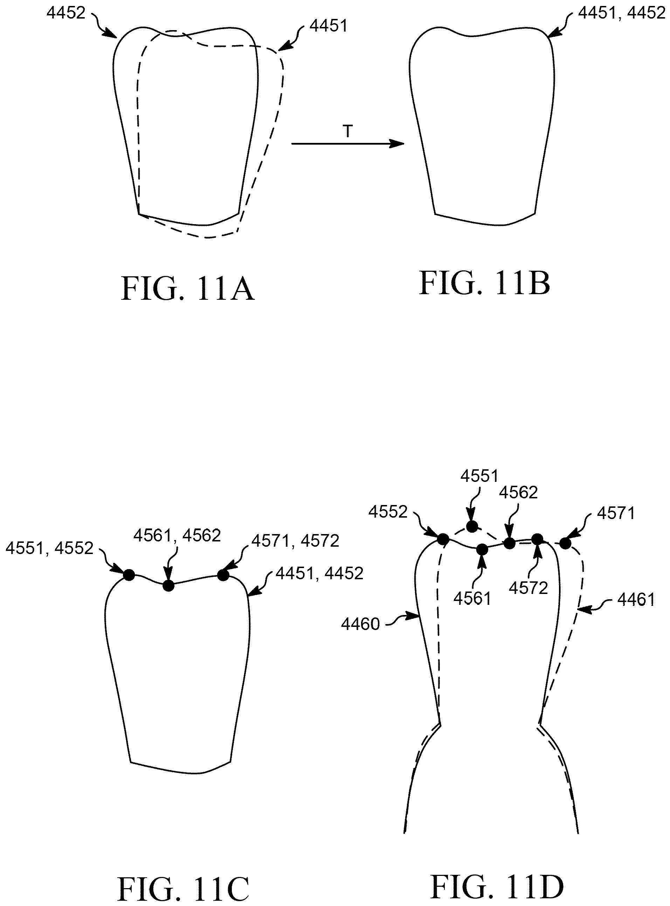

FIG. 11A shows cross sections of a first 3D tooth model 4451 and a second 3D tooth model 4452 segmented from a first and a second digital 3D representation, respectively. The first 3D digital scan/representation can for example represent the subject/patient's teeth at the onset of an orthodontic treatment and the second 3D digital scan/representation at some point during the treatment - two different points in time. A transformation T that may align the first 3D tooth model 4451 with the second 3D tooth model 4452 may be determined and/or applied to the first 3D tooth model 4451 to provide that the two tooth models 4451, 4452 may be locally aligned as illustrated in FIG. 11B.

In FIG. 11C three portions 4551, 4561 and 4571 are selected on the first 3D tooth model 4451. Since the first and second 3D tooth models 4451, 4452 are locally aligned the anatomically corresponding portions 4552, 4562 and 4572 can readily and precisely be identified on the second 3D tooth model 4452. In FIG. 11D portions 4551, 4561 and 4571 are marked on corresponding portions of the first 3D digital scan/representation 4460 and portions 4552, 4562 and 4572 are marked on the corresponding portions of the second 3D digital scan/representation 4461. The first and second 3D digital scan/representations 4460, 4461 may be globally aligned based, for example, on the other teeth of the same quadrant or the same arch (not shown). The anatomical correct distance between the marked regions can then be determined and based on these distances a measure for the movement of the tooth between the first and second point in time can be derived.

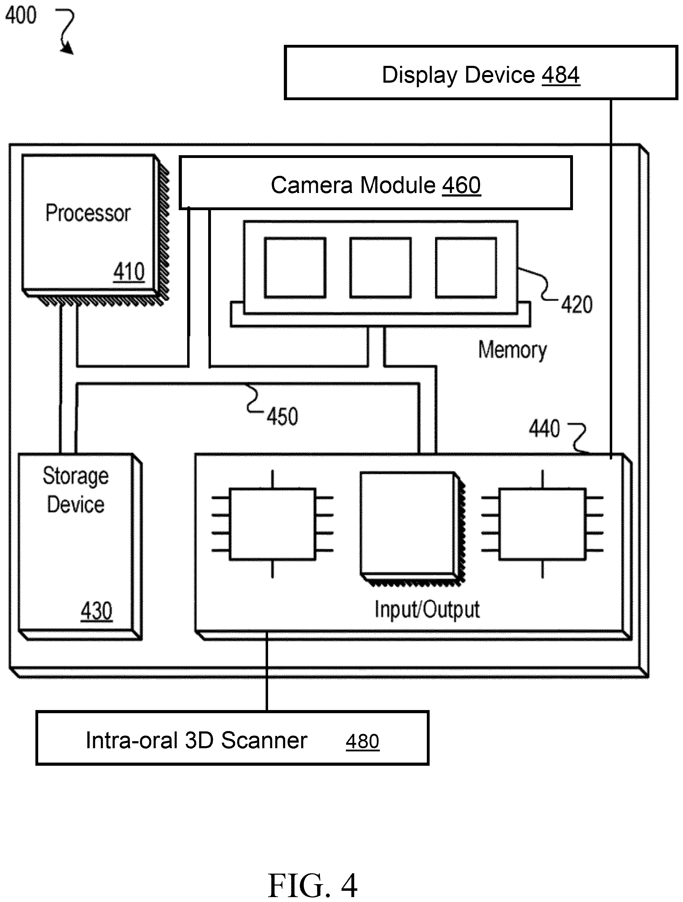

FIG. 4 is a block diagram of a hardware configuration of an example device that may function as a process control device/logic controller for example on/via which any of the ML algorithms/models/neural network processing/techniques disclosed herein may be conducted. The hardware configuration 400 may be in wired and/or wireless communication, and/or in Internet/cloud communication, with any of the scanner systems/devices described herein. The hardware configuration 400 may be operable to facilitate delivery of information from an internal server of a device. The hardware configuration 400 can include a processor 410, a memory 420, a storage device 430, and/or an input/output device 440. One or more of the components 410, 420, 430, and 440 can, for example, be interconnected using a system bus 450. The processor 410 can process instructions for execution within the hardware configuration 400. The processor 410 can be a single-threaded processor or the processor 410 can be a multi-threaded processor. The processor 410 can be capable of processing instructions stored in the memory 420 and/or on the storage device 430.

The memory 420 can store information within the hardware configuration 400. The memory 420 can be a computer-readable medium (CRM), for example, a non-transitory CRM. The memory 420 can be a volatile memory unit, and/or can be a non-volatile memory unit.

The storage device 430 can be capable of providing mass storage for the hardware configuration 400. The storage device 430 can be a computer-readable medium (CRM), for example, a non-transitory CRM. The storage device 430 can, for example, include a hard disk device, an optical disk device, flash memory and/or some other large capacity storage device. The storage device 430 can be a device external to the hardware configuration 400.

The input/output device 440 may provide input/output operations for the hardware configuration 400. The input/output device 440 (e.g., a transceiver device) can include one or more of a network interface device (e.g., an Ethernet card), a serial communication device *(e.g.,* an RS-232 port), one or more universal serial bus (USB) interfaces (e.g., a USB 2.0 port) and/or a wireless interface device (e.g., an 802.11 card). The input/output device can include driver devices configured to send communications to, and/or receive communications from one or more networks. The input/output device 440 may be in communication with at least one display device 484. The display device 484 may display one or more of the MGI values (e.g., a MGI value, a gingival index (GI) value, and/or another index value related to a gingival condition) and/or the pocket depth values, for example as at least part of the various digital representations of the oral cavity elements described herein.

The input/output device 400 may be in communication with one or more input/output modules (not shown) that may be proximate to the hardware configuration 400 and/or may be remote from the hardware configuration 400. The one or more output modules may provide input/output functionality in the digital signal form, discrete signal form, TTL form, analog signal form, serial communication protocol, fieldbus protocol communication and/or other open or proprietary communication protocol, and/or the like.

The camera module 460 may provide digital video input/output capability for the hardware configuration 400. The camera module 460 may communicate with any of the elements of the hardware configuration 400, perhaps for example via system bus 450. The camera module 460 may capture digital images and/or may scan images of various kinds, such as Universal Product Code (UPC) codes and/or Quick Response (QR) codes, for example, among other images as described herein. For example, a dental patient's record may be accessible via a QR code, or the like. The camera module 460 may scan the patient dental record QR code to access a patient's dental record such that one or more of the gingivitis analysis and/or the periodontal pocket depth analysis may be (e.g., electronically) added to the patient's dental record.

The camera module 460 may include at least one microphone device and/or at least one speaker device (not shown). The camera module 460 may be in wired and/or wireless communication with the hardware configuration 400. In one or more scenarios, the camera module 460 may be external to the hardware configuration 400. In one or more scenarios, the camera module 460 may be internal to the hardware configuration 400.

An intra-oral 3D scanner (IOS) 480 may be in wired and/or wireless communication with the hardware configuration 400. The IOS 480 may be any one of an intra-oral 3D scanner capable of/configured to provide at least the 3D digital scan data of a subject's oral cavity as described herein.

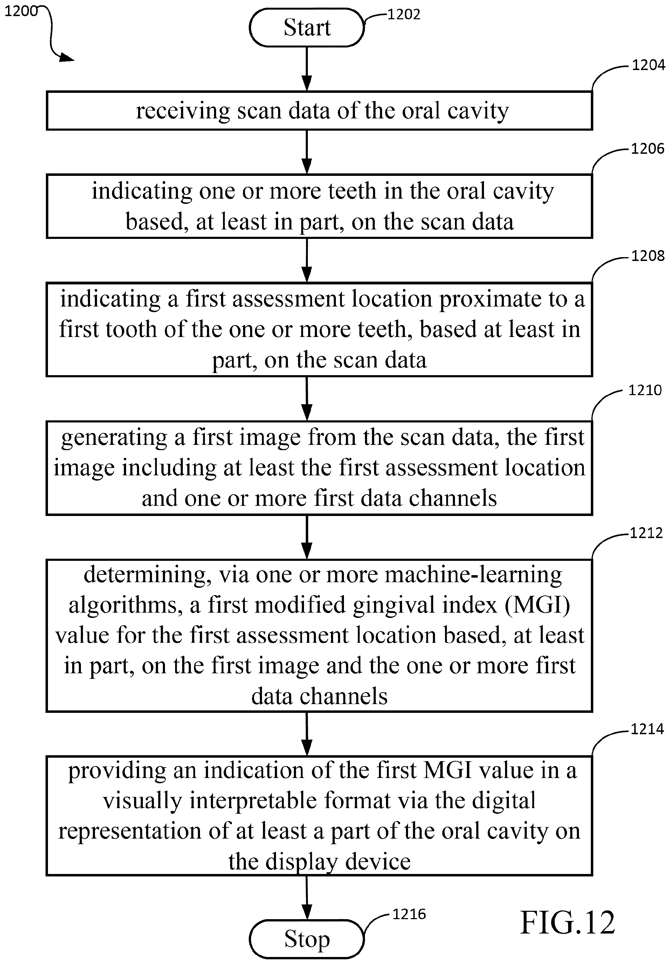

In view of FIG. 1 to FIG. 11D, referring now to FIG. 12, a diagram 1200 illustrates an example technique/method/process for detection and/or display of an indication of a gingivitis condition of a subject's oral cavity via a digital representation of the oral cavity. The method may be performed by a scanner system/device and/or an imaging analysis device, and/or a cloud computing device, among other devices. The scanner system/device and/or imagining analysis device may be in communication with at least one virtual camera, a digital camera, and/or a display device. At 1202, the process may start or restart.

At 1204, one or more techniques may comprise receiving scan data of the oral cavity (e.g., 3D scan data). At 1206, one or more technique may comprise indicating one or more teeth in the oral cavity based, at least in part, on the scan data.

At 1208, one or more techniques may comprise indicating a first assessment location proximate to a first tooth of the one or more teeth, based at least in part, on the scan data. At 1210, one or more techniques may comprise generating a first image from the scan data, the first image including at least the first assessment location and one or more first data channels.

At 1212, one or more techniques may comprise determining, via one or more machine-learning algorithms, a first modified gingival index (MGI) value (e.g., a MGI value, a gingival index (GI) value, and/or another index value related to a gingival condition) for the first assessment location based, at least in part, on the first image and the one or more first data channels.

At 1214, one or more techniques may comprise providing an indication of the first MGI value (e.g., a MGI value, a gingival index (GI) value, and/or another index value related to a gingival condition) in a visually interpretable format via the digital representation of at least a part of the oral cavity on the display device. At 1216, the process may stop or restart.

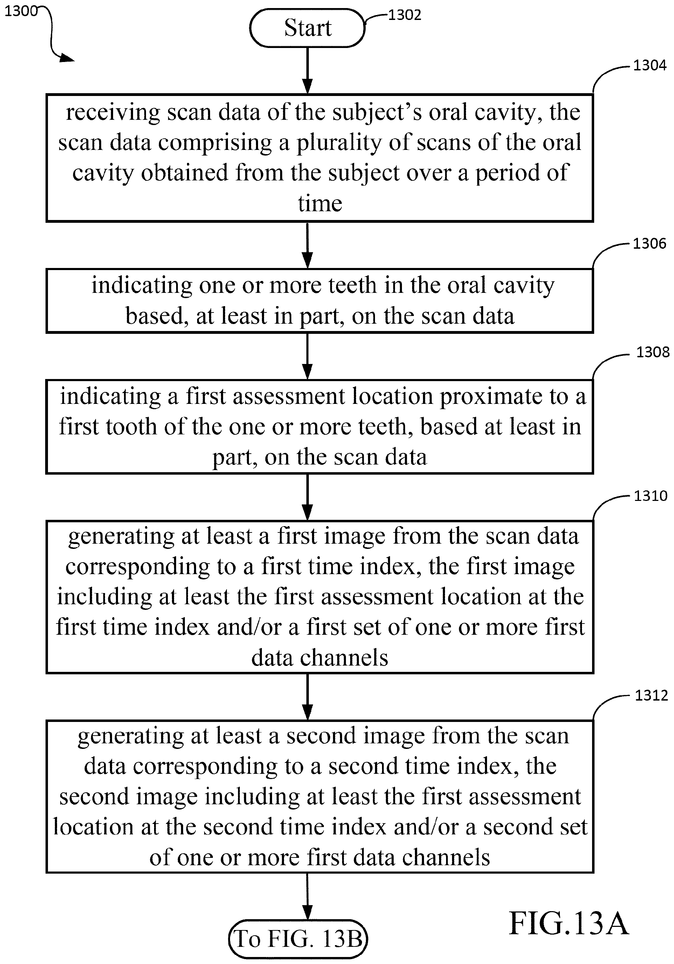

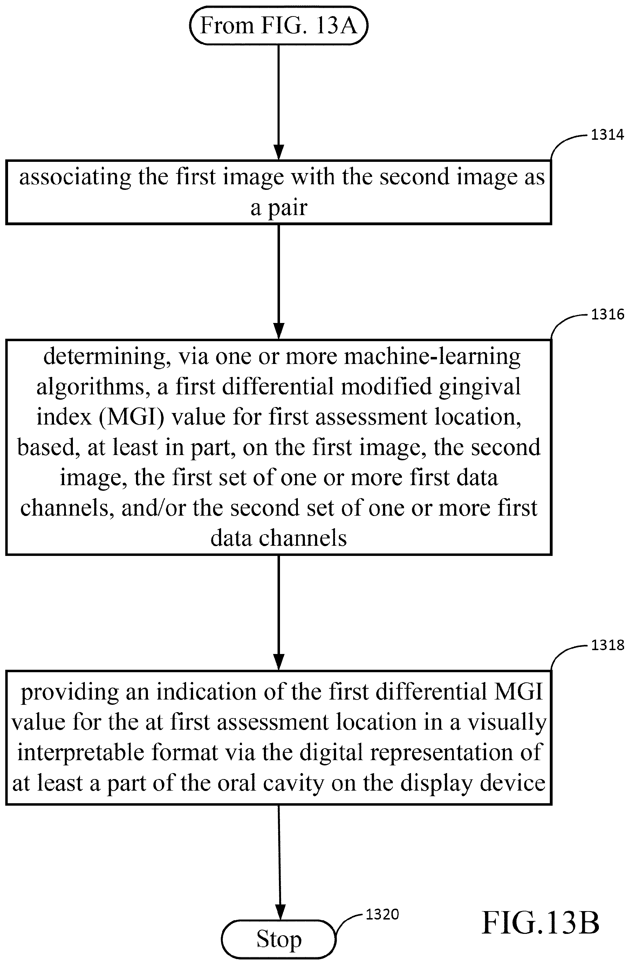

In view of FIG. 1 to FIG. 11D, referring now to FIG. 13A and FIG. 13B, a diagram 1300 illustrates an example technique/method/process for detection and/or display of an indication of a gingivitis condition of a subject's oral cavity via a digital representation of the oral cavity. The method may be performed by a scanner system/device and/or an imaging analysis device, and/or a cloud computing device, among other devices. The scanner system/device and/or imagining analysis device may be in communication with at least one virtual camera, a digital camera, and/or a display device. At 1302, the process may start or restart.

At 1304, one or more techniques may comprise receiving scan data of the subject's oral cavity. The scan data may comprise a plurality of scans of the oral cavity obtained from the subject over a period of time. At 1306, one or more techniques may comprise indicating one or more teeth in the oral cavity based, at least in part, on the scan data.

At 1308, one or more techniques may comprise indicating a first assessment location proximate to a first tooth of the one or more teeth, based at least in part, on the scan data. At 1310, one or more techniques may comprise generating at least a first image from the scan data corresponding to a first time index. The first image may include at least the first assessment location at the first time index and/or a first set of one or more first data channels.

At 1312, one or more techniques may comprise generating at least a second image from the scan data corresponding to a second time index. The second image may include at least the first assessment location at the second time index and/or a second set of one or more first data channels.

At 1314, one or more techniques may comprise associating the first image with the second image as a pair. At 1316, one or more techniques may comprise determining, via one or more machine-learning algorithms, a first differential modified gingival index (MGI) value (e.g., a first differential MGI value, a first differential gingival index (GI) value, and/or another first differential index value related to a gingival condition) for first assessment location, based, at least in part, on the first image, the second image, the first set of one or more first data channels, and/or the second set of one or more first data channels.

At 1318, one or more techniques may comprise providing an indication of the first differential MGI value (e.g., a first differential MGI value, a first differential gingival index (GI) value, and/or another first differential index value related to a gingival condition) for the at first assessment location in a visually interpretable format via the digital representation of at least a part of the oral cavity on the display device. At 1320, the process may stop or restart.

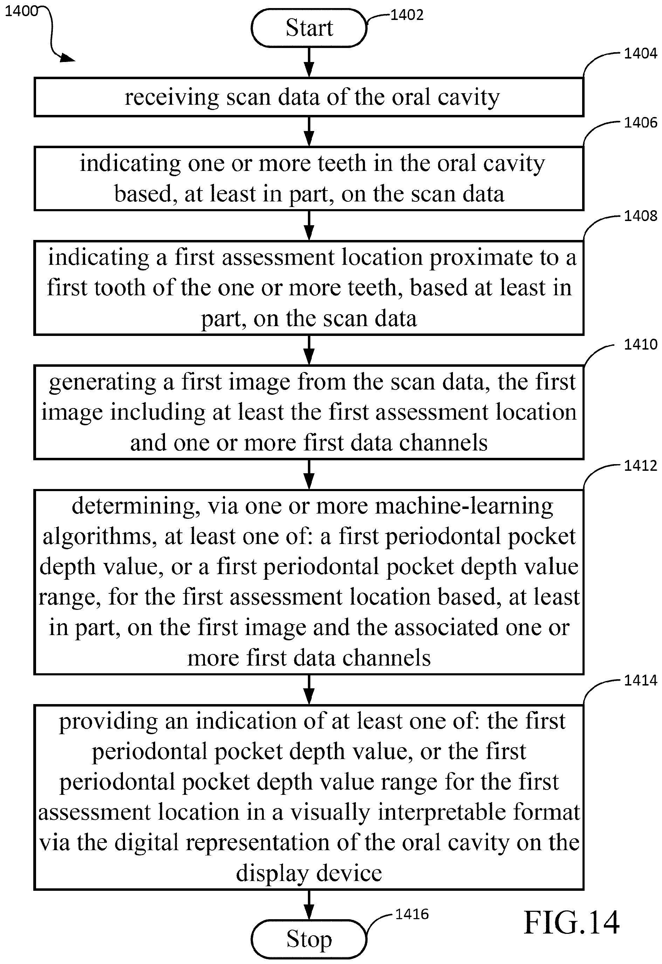

In view of FIG. 1 to FIG. 11D, referring now to FIG. 14, a diagram 1400 illustrates an example technique/method/process for detection and/or display of an indication of a periodontal pocket depth of a subject's oral cavity via a digital representation of the oral cavity. The method may be performed by a scanner system/device and/or an imaging analysis device, and/or a cloud computing device, among other devices. The scanner system/device and/or imagining analysis device may be in communication with at least one virtual camera, a digital camera, and/or a display device. At 1402, the process may start or restart.

At 1404, one or more techniques may comprise receiving scan data of the oral cavity. At 1406, one or more techniques may comprise indicating one or more teeth in the oral cavity based, at least in part, on the scan data.

At 1408, one or more techniques may comprise indicating a first assessment location proximate to a first tooth of the one or more teeth, based at least in part, on the scan data. At 1410, one or more techniques may comprise generating a first image from the scan data, the first image including at least the first assessment location and one or more first data channels.

At 1412, one or more techniques may comprise determining, via one or more machine-learning algorithms, at least one of: a first periodontal pocket depth value, or a first periodontal pocket depth value range, for the first assessment location based, at least in part, on the first image and the associated one or more first data channels.

At 1414, one or more techniques may comprise providing an indication of at least one of: the first periodontal pocket depth value, or the first periodontal pocket depth value range for the first assessment location in a visually interpretable format via the digital representation of the oral cavity on the display device. At 1416, the process may stop or restart.

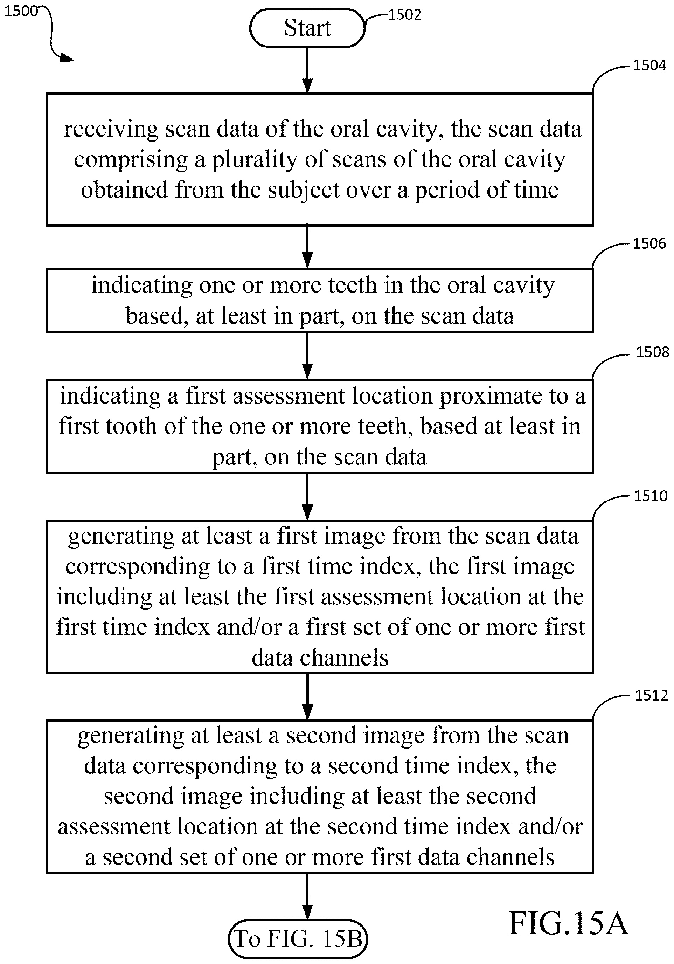

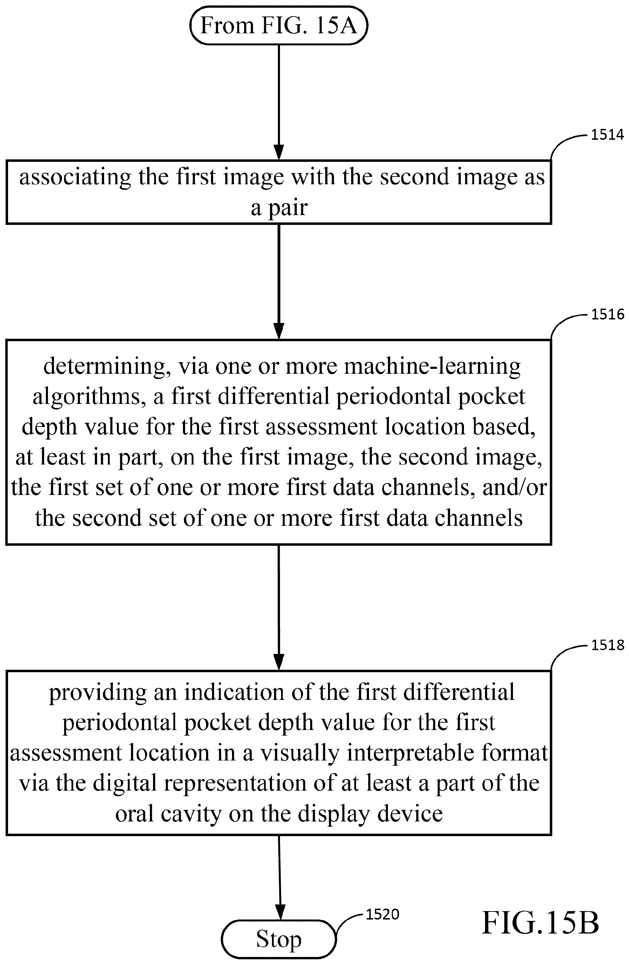

In view of FIG. 1 to FIG. 11D, referring now to FIG. 15A and FIG. 15B, a diagram 1500 illustrates an example technique/method/process for detection and/or display of an indication of a periodontal pocket depth of a subject's oral cavity via a digital representation of the oral cavity. The method may be performed by a scanner system/device and/or an imaging analysis device, and/or a cloud computing device, among other devices. The scanner system/device and/or imagining analysis device may be in communication with at least one virtual camera, a digital camera, and/or a display device. At 1502, the process may start or restart.

At 1504, one or more techniques may comprise receiving scan data of the oral cavity. The scan data may comprise a plurality of scans of the oral cavity obtained from the subject over a period of time. At 1506, one or more techniques may comprise indicating one or more teeth in the oral cavity based, at least in part, on the scan data.

At 1508, one or more techniques may comprise indicating a first assessment location proximate to a first tooth of the one or more teeth, based at least in part, on the scan data. At 1510, one or more techniques may comprise generating at least a first image from the scan data corresponding to a first time index. The first image may include at least the first assessment location at the first time index and/or a first set of one or more first data channels.

At 1512, one or more techniques may comprise generating at least a second image from the scan data corresponding to a second time index. The second image may include at least the second assessment location at the second time index and/or a second set of one or more first data channels. At 1514, one or more techniques may comprise associating the first image with the second image as a pair.

At 1516, one or more techniques may comprise determining, via one or more machine-learning algorithms, a first differential periodontal pocket depth value for the first assessment location based, at least in part, on the first image, the second image, the first set of one or more first data channels, and/or the second set of one or more first data channels.

At 1518, one or more techniques may comprise providing an indication of the first differential periodontal pocket depth value for the first assessment location in a visually interpretable format via the digital representation of at least a part of the oral cavity on the display device. At 1520, the process may stop or restart.

In view of FIG. 1 to FIG. 11D, in one or more scenarios, a scanner system, and/or techniques performed thereby, may be configured for detection and/or display of an indication of a gingivitis condition of a subject's oral cavity via a digital representation of the oral cavity. The system may comprise a memory, a display device, and/or a processor. The processor may be configured to receive scan data of the oral cavity. The processor may be configured to indicate one or more teeth in the oral cavity based, at least in part, on the scan data. The processor may be configured to indicate a first assessment location proximate to a first tooth of the one or more teeth, based at least in part, on the scan data.

The processor may be configured to generate a first image from the scan data. The first image may include at least the first assessment location and/or one or more first data channels. The one or more first data channels may comprise color data and/or topological information corresponding to the first assessment location.

The processor may be configured to determine, perhaps via one or more machine-learning algorithms, a first modified gingival index (MGI) value for the first assessment location based, at least in part, on the first image and/or the one or more first data channels. The processor may be configured to provide an indication of the first MGI value (e.g., a first modified gingival index (MGI) value, a first gingival index (GI), and/or another first index related to a gingival condition) in a visually interpretable format via the digital representation of at least a part of the oral cavity on the display device.

In one or more scenarios, the topological information may comprise information obtained from the scan data. The topological information may comprise at least one of: one or more facet normals, one or more oral cavity depth measurements, and/or oral cavity surface curvature data, or other topological information/data as described herein.

In one or more scenarios, the scan data may comprise at least a three-dimensional (3D) representation of the oral cavity. In one or more scenarios, the processor may be further configured such that the first image may comprise at least a two-dimensional (2D) image.

In one or more scenarios, the processor may be configured such that the digital representation of the at least part of the oral cavity may be at least one of: a two-dimensional (2D) digital representation of the at least part of the oral cavity, and/or a three-dimensional (3D) digital representation of the at least part of the oral cavity.

In one or more scenarios, the scanner system may comprise an intra-oral scanner. The intra-oral scanner may comprise at least one light emitting device. The intra-oral scanner may comprise an imaging sensor. The imaging sensor may be configured to capture reflected light of a dental object in the oral cavity. The imaging sensor may be configured to provide the scan data of the oral cavity, based at least in part, on the reflected light.

In one or more scenarios, the processor may be configured such that the one or more first data channels may comprise at least six first data channels. In one or more scenarios, the processor may be configured such that the color data may comprise a color space definition of the first assessment location. In one or more scenarios, the processor may be configured such that the color space definition of the first assessment location may comprise at least one of: a red color, a green color, and/or a blue color. In one or more scenarios, the color data may comprise one or more fluorescence colors.

In one or more scenarios, the processor may be configured such that the one or more facet normals are communicated as camera image coordinates and/or may comprise one or more of: a Nx component, a Ny component, and/or a Nz component.

In one or more scenarios, the processor may be configured such that at least one of: the Nx component, the Ny component, and/or the Nz component may identify the first assessment location in the color space definition of the first assessment location. In one or more scenarios, the first assessment location may comprise at least some of a gingiva area of the oral cavity.

In one or more scenarios, the processor may be configured to determine a tooth number for each of the one or more teeth.

In one or more scenarios, the processor may be configured such that the one or more machine-learning algorithms may determine the first MGI value (e.g., a first modified gingival index (MGI) value, a first gingival index (GI), and/or another first index related to a gingival condition) based, at least in part, on one or more of: an outer shape of soft tissue, a gingival color, a shape of a gingival margin, a position of the gingival margin, a position of a cemento-enamel junction (CEJ), and/or a curvature of at least the first assessment location.

In one or more scenarios, the processor may be configured to indicate a first side and a second side of the first tooth, based at least in part, on the scan data. The processor may be configured to indicate a second assessment location proximate to the first tooth, based at least in part, on the scan data. The processor may be configured to indicate a third assessment location proximate to the first tooth, based at least in part, on the scan data.

The processor may be configured to generate a second image from the scan data. The second image may include at least the second assessment location and/or one or more second data channels. The one or more second data channels may comprise color data and/or topological information corresponding to the second assessment location.

The processor may be configured to generate a third image from the scan data. The third image may include at least the third assessment location and/or one or more third data channels. The one or more third data channels may comprise color data and/or topological information corresponding to the third assessment location.

In one or more scenarios, the processor may be configured such that the first assessment location is at least one of: proximate to the first side of the first tooth along with the second assessment location and the third assessment location; proximate to the first side of the first tooth along with the second assessment location, where the third assessment location may be proximate to the second side of the first tooth; and/or proximate to the first side of the first tooth, where the second assessment location and the third assessment location may be proximate to the second side of the first tooth.

In one or more scenarios, the processor may be configured to indicate a fourth assessment location proximate to the first tooth, based at least in part, on the scan data. The processor may be configured to indicate a fifth assessment location proximate to the first tooth, based at least in part, on the scan data. The processor may be configured to indicate a sixth assessment location proximate to the first tooth, based at least in part, on the scan data.

The processor may be configured to generate a fourth image from the scan data. The fourth image may include at least the fourth assessment location and/or one or more fourth data channels. The one or more fourth data channels may comprise color data and/or topological information corresponding to the fourth assessment location. The processor may be configured to generate a fifth image from the scan data. The fifth image may include at least the fifth assessment location and/or one or more fifth data channels. The one or more fifth data channels may comprise color data and/or topological information corresponding to the fifth assessment location.

The processor may be configured to generate a sixth image from the scan data. The sixth image may include at least the sixth assessment location and/or one or more sixth data channels. The one or more sixth data channels may comprise color data and/or topological information corresponding to the sixth assessment location.

In one or more scenarios, the processor may be configured such that the fourth assessment location may be at least one of: proximate to the second side of the first tooth along with the fifth assessment location and the sixth assessment location; proximate to the second side of the first tooth along with the fifth assessment location, where the sixth assessment location may be proximate to the first side of the first tooth; and/or proximate to the second side of the first tooth, where the fifth assessment location and the sixth assessment location may be proximate to the first side of the first tooth.

In one or more scenarios, the processor may be configured such that the visually interpretable format for the indication of the first MGI value (e.g., a first modified gingival index (MGI) value, a first gingival index (GI), and/or another first index related to a gingival condition) may comprise a numerical representation of the first MGI value (e.g., a first modified gingival index (MGI) value, a first gingival index (GI), and/or another first index related to a gingival condition) imposed proximate to the first assessment location on the digital representation of the at least part of the oral cavity.

The processor may be configured such that the visually interpretable format for the indication of the first MGI value (e.g., a first modified gingival index (MGI) value, a first gingival index (GI), and/or another first index related to a gingival condition) may comprise a representation of bleeding intensity corresponding to the first MGI value (e.g., a first modified gingival index (MGI) value, a first gingival index (GI), and/or another first index related to a gingival condition) imposed proximate to the first assessment location on the digital representation of the at least part of the oral cavity. The bleeding intensity representation may vary from significant bleeding corresponding to a relatively high first MGI value (e.g., a first modified gingival index (MGI) value, a first gingival index (GI), and/or another first index related to a gingival condition) to a non-bleeding condition corresponding to a relatively low first MGI value (e.g., a first modified gingival index (MGI) value, a first gingival index (GI), and/or another first index related to a gingival condition).

The processor may be configured such that the visually interpretable format for the indication of the first MGI value (e.g., a first modified gingival index (MGI) value, a first gingival index (GI), and/or another first index related to a gingival condition) may comprise a heat map shading differentiation corresponding to the first MGI value (e.g., a first modified gingival index (MGI) value, a first gingival index (GI), and/or another first index related to a gingival condition) imposed proximate to the first assessment location on the digital representation of the at least part of the oral cavity. The heat map color differentiation may vary from dark shading corresponding to a relatively high first MGI value (e.g., a first modified gingival index (MGI) value, a first gingival index (GI), and/or another first index related to a gingival condition) to a light shading corresponding to a relatively low first MGI value (e.g., a first modified gingival index (MGI) value, a first gingival index (GI), and/or another first index related to a gingival condition).

In one or more scenarios, the processor may be configured to determine, perhaps via the one or more machine-learning algorithms, one or more of: a second MGI value (e.g., a second modified gingival index (MGI) value, a second gingival index (GI), and/or another second index related to a gingival condition) for the second assessment location based, at least in part, on the second image and the one or more second data channels; a third MGI value (e.g., a third modified gingival index (MGI) value, a third gingival index (GI), and/or another third index related to a gingival condition) for the third assessment location based, at least in part, on the third image and the one or more third data channels; a fourth MGI value (e.g., a fourth modified gingival index (MGI) value, a fourth gingival index (GI), and/or another fourth index related to a gingival condition) for the fourth assessment location based, at least in part, on the fourth image and the one or more fourth data channels; a fifth MGI value (e.g., a fifth modified gingival index (MGI) value, a fifth gingival index (GI), and/or another fifth index related to a gingival condition) for the fifth assessment location based, at least in part, on the fifth image and the one or more fifth data channels; and/or a sixth MGI value (e.g., a sixth modified gingival index (MGI) value, a sixth gingival index (GI), and/or another sixth index related to a gingival condition) for the sixth assessment location based, at least in part, on the sixth image and the one or more sixth data channels.

The processor may be configured to provide an indication of at least one of: the second MGI value, the third MGI value, the fourth MGI value, the fifth MGI value, and/or the sixth MGI value, in a visually interpretable format via the digital representation of at least a part of the oral cavity on the display device.

In one or more scenarios, the processor may be configured to provide an indication of one or more of: the second MGI value, the third MGI value, the fourth MGI value, the fifth MGI value, and/or the sixth MGI value in a visually interpretable format via the digital representation of at least a part of the oral cavity on the display device, via a numerical representation of at least one of: the second MGI value, the third MGI value, the fourth MGI value, the fifth MGI value, and/or the sixth MGI value that may be imposed proximate to the assessment location corresponding to the respective MGI value (e.g., a respective modified gingival index (MGI) value, a respective gingival index (GI), and/or another respective index related to a gingival condition) on the digital representation of the at least part of the oral cavity.

In one or more scenarios, the processor may be configured to provide an indication of one or more of: the second MGI value, the third MGI value, the fourth MGI value, the fifth MGI value, and/or the sixth MGI value in a visually interpretable format via the digital representation of at least a part of the oral cavity on the display device, via a representation of bleeding intensity corresponding to one or more of: the second MGI value, the third MGI value, the fourth MGI value, the fifth MGI value, or the sixth MGI value, that may be imposed proximate to the assessment location corresponding to the respective MGI value (e.g., a respective modified gingival index (MGI) value, a respective gingival index (GI), and/or another respective index related to a gingival condition) on the digital representation of the at least part of the oral cavity. The bleeding intensity representation for the respective MGI values may vary from significant bleeding corresponding to a relatively high MGI value (e.g., a respective modified gingival index (MGI) value, a respective gingival index (GI), and/or another respective index related to a gingival condition) to a non-bleeding condition corresponding to a relatively low MGI value (e.g., a respective modified gingival index (MGI) value, a respective gingival index (GI), and/or another respective index related to a gingival condition).

In one or more scenarios, the processor may be configured to provide an indication of one or more of: the second MGI value, the third MGI value, the fourth MGI value, the fifth MGI value, and/or the sixth MGI value in a visually interpretable format via the digital representation of at least a part of the oral cavity on the display device, via a heat map shading differentiation corresponding to one or more of: the second MGI value, the third MGI value, the fourth MGI value, the fifth MGI value, and/or the sixth MGI value, that may be imposed proximate to the assessment location corresponding to the respective MGI value on the digital representation of the at least part of the oral cavity. The heat map color differentiation for the respective MGI values (e.g., a respective modified gingival index (MGI) value, a respective gingival index (GI), and/or another respective index related to a gingival condition) may be from dark shading corresponding to a relatively high MGI value to a light shading corresponding to a relatively low MGI value.

In one or more scenarios, the processor may be configured such that any MGI value may be determined as an integer value from a range of 0 to 4.

In one or more scenarios, the processor may be configured such that the one or more machine-learning algorithms are adjustable into one or more modes. The one or mode modes may comprise an operational mode and/or a learning mode. The processor may be configured to receive a plurality of calibration images of oral cavity tissue areas. One or more, or each of the plurality of calibration images may include one or more calibration data channels.

The processor may be configured to receive a predetermined MGI value (e.g., a predetermined modified gingival index (MGI) value, a predetermined gingival index (GI), and/or another predetermined index related to a gingival condition) for each of the plurality of calibration images. The processor may be configured to adjust the one or more machine-learning algorithms into the learning mode. The processor may be configured to associate, by the one or more machine-learning algorithms in the learning mode, the predetermined MGI value (e.g., a predetermined modified gingival index (MGI) value, a predetermined gingival index (GI), and/or another predetermined index related to a gingival condition) for each of the plurality of calibration images and/or the corresponding one or more calibration data channels.

The processor may be configured to perform a calibration of the one or more machine-learning algorithms based, at least in part, on the association of the predetermined MGI value (e.g., a predetermined modified gingival index (MGI) value, a predetermined gingival index (GI), and/or another predetermined index related to a gingival condition) with the plurality of calibration images and/or the corresponding one or more calibration data channels. The calibration may include an iterative update of one or more parameters to minimize at least one of: an objective function, and/or a loss function, of the one or more machine-learning algorithms.

In one or more scenarios, a scanner system, and/or techniques performed thereby, may be configured for detection and/or display of an indication of a gingivitis condition of a subject's oral cavity via a digital representation of the oral cavity. The system may comprise a memory, a display device, and/or a processor. The processor may be configured to receive scan data of the subject's oral cavity. The scan data may comprise a plurality of scans of the oral cavity obtained from the subject over a period of time. The processor may be configured to indicate one or more teeth in the oral cavity based, at least in part, on the scan data.

The processor may be configured to indicate a first assessment location proximate to a first tooth of the one or more teeth, based at least in part, on the scan data. The processor may be configured to generate at least a first image from the scan data corresponding to a first time index. The first image may include at least the first assessment location at the first time index and/or a first set of one or more first data channels. The first set of one or more first data channels may comprise color data and/or topological information corresponding to the first assessment location at the first time index.

The processor may be configured to generate at least a second image from the scan data corresponding to a second time index. The second image may include at least the first assessment location at the second time index and/or a second set of one or more first data channels. The second set of one or more first data channels may comprise color data and/or topological information corresponding to the first assessment location at the second time index.

The processor may be configured to associate the first image with the second image as a pair. The processor may be configured to determine, perhaps via one or more machine-learning algorithms, a first differential modified gingival index (MGI) value (e.g., a first differential modified gingival index (MGI) value, a first differential gingival index (GI), and/or another first differential index related to a gingival condition) for first assessment location, based, at least in part, on the first image, the second image, the first set of one or more first data channels, and/or the second set of one or more first data channels.

The processor may be configured to provide an indication of the first differential MGI value (e.g., a first differential modified gingival index (MGI) value, a first differential gingival index (GI), and/or another first differential index related to a gingival condition) for the first assessment location in a visually interpretable format via the digital representation of at least a part of the oral cavity on the display device.

In one or more scenarios, the topological information corresponding to the first assessment location at the first time index may comprise information obtained from the scan data. The topological information corresponding to the first assessment location at the first time index may comprise at least one of: one or more facet normal corresponding to the first assessment location at the first time index, one or more oral cavity depth measurements corresponding to the first assessment location at the first time index, and/or oral cavity surface curvature data corresponding to the first assessment location at the first time index.

In one or more scenarios, the topological information corresponding to the first assessment location at the second time index may comprise information obtained from the scan data. The topological information corresponding to the first assessment location at the second time index may comprise at least one of: one or more facet normal corresponding to the first assessment location at the second time index, one or more oral cavity depth measurements corresponding to the first assessment location at the second time index, and/or oral cavity surface curvature data corresponding to the first assessment location at the second time index.

In one or more scenarios, the scan data may comprise at least a three-dimensional (3D) representation of the oral cavity over the period of time. In one or more scenarios, the processor may be configured such that at least one of: the first image, and/or the second image, may comprise at least a two-dimensional (2D) image.

In one or more scenarios, the processor may be configured such that the digital representation of the at least part of the oral cavity is at least one of: a two-dimensional (2D) digital representation of the at least part of the oral cavity, and/or a three-dimensional (3D) digital representation of the at least part of the oral cavity.

In one or more scenarios, the system may comprise an intra-oral scanner. The intra-oral scanner may comprise at least one light emitting device. The intra-oral scanner may comprise an imaging sensor. The imaging sensor may be configured to capture reflected light of a dental object in the oral cavity. The imaging sensor may be configured to provide the scan data of the oral cavity, based at least in part, on the reflected light.

In one or more scenarios, the processor may be configured such that the first set of one or more first data channels may comprise a first group of at least six first data channels, and/or the second set of one or more data channels may comprise a second group of at least six first data channels.

In one or more scenarios, the processor may be configured such that the color data of the first set of one or more first data channels may comprise a color space definition of the first assessment location at the first time index, and/or the color data of the second set of one or more first data channels may comprise a color space definition of the first assessment location at the second time index.

In one or more scenarios, the processor may be configured such that at least one of: the color space definition of the first assessment location at the first time index, and/or the color space definition of the first assessment location at the second time index, may comprise at least one of: a red color, a green color, and/or a blue color. In one or more scenarios, at least one of: the color data of the first assessment location at the first time index, and/or the color data of the first assessment location at the second time index, may comprise one or more fluorescence colors.

In one or more scenarios, the processor may be configured such that the one or more facet normals associated with the first assessment location at the first time index may be communicated as camera image coordinates and/or may comprise one or more of: a Nx component, a Ny component, and/or a Nz component.

In one or more scenarios, the processor may be configured such that the one or more facet normals associated with the first assessment location at the second time index may be communicated as camera image coordinates and/or may comprise one or more of: a Nx component, a Ny component, and/or a Nz component.

In one or more scenarios, the processor may be configured such that at least one of: the Nx component, the Ny component, and/or the Nz component, corresponding to the first assessment location at the first time index may identify the first assessment location in the color space definition of the first assessment location at the first time index.

The processor may be configured such that at least one of the Nx component, the Ny component, and/or the Nz component corresponding to the first assessment location at the second time index may identify the first assessment location in the color space definition of the first assessment location at the second time index.

In one or more scenarios, the first assessment location may comprise at least some of a gingiva area of the oral cavity. In one or more scenarios, the processor may be configured to determine a tooth number of each of the one or more teeth.

In one or more scenarios, the processor may be configured such that the one or more machine-learning algorithms may determine the first differential MGI value (e.g., a first differential modified gingival index (MGI) value, a first differential gingival index (GI), and/or another first differential index related to a gingival condition) based, at least in part, on one or more of: an outer shape of soft tissue, a gingival color, a shape of a gingival margin, a position of the gingival margin, a position of a cemento-enamel junction (CEJ), a curvature, a volumetric change in the oral cavity, a shape change in the oral cavity, a surface shape change in the oral cavity, and/or a color change of the soft tissue of the first assessment location.

In one or more scenarios, the processor may be configured to indicate a first side and a second side of the first tooth, based at least in part, on the scan data. The processor may be configured to indicate a second assessment location proximate to the first tooth, based at least in part, on the scan data. The processor may be configured to indicate a third assessment location proximate to the first tooth, based at least in part, on the scan data. The processor may be configured to generate a third image and a fourth image from the scan data. The processor may be configured to generate a fifth image and a sixth image from the scan data.

The processor may be configured to associate the third image with the fourth image as a pair. The third image and the fourth image may include at least the second assessment location. The third image may be associated with the second assessment location at a first time index. The fourth image may be associated with the second assessment location at a second time index.

The processor may be configured to associate the fifth image with the sixth image as a pair. The fifth image and the sixth image may include at least the third assessment location. The fifth image may be associated with the third assessment location at a first time index. The sixth image may be associated with the third assessment location at a second time index. The third image may include a first set of one or more second data channels that may comprise color data and/or topological information corresponding to the second assessment location at the first time index. The fourth image may include a second set of the one or more second data channels that may comprise color data and/or topological information corresponding to the second assessment location at the second time index.

The fifth image may include a first set of one or more third data channels that may comprise color data and/or topological information corresponding to the third assessment location at the first time index. The sixth image may include a second set of the one or more third data channels that may comprise color data and/or topological information corresponding to the third assessment location at the second time index.

In one or more scenarios, the processor may be configured such that the first assessment location is at least one of: proximate to the first side of the first tooth along with the second assessment location and the third assessment location; proximate to the first side of the first tooth along with the second assessment location, where the third assessment location may be proximate to the second side of the first tooth; and/or proximate to the first side of the first tooth, where the second assessment location and the third assessment location may be proximate to the second side of the first tooth.

In one or more scenarios, the processor may be configured to indicate a fourth assessment location proximate to the first tooth, based at least in part, on the scan data. The processor may be configured to indicate a fifth assessment location proximate to the first tooth, based at least in part, on the scan data. The processor may be configured to indicate a sixth assessment location proximate to the first tooth, based at least in part, on the scan data.

The processor may be configured to generate a seventh image and an eighth image from the scan data. The processor may be configured to generate a ninth image and a tenth image from the scan data. The processor may be configured to generate an eleventh image and a twelfth image from the scan data.

The processor may be configured to associate the seventh image with the eighth image as a pair. The seventh image and the eighth image may include at least the fourth assessment location. The seventh image may be associated with the fourth assessment location at a first time index. The eighth image may be associated with the fourth assessment location at a second time index.

The processor may be configured to associate the ninth image with the tenth image as a pair. The ninth image and the tenth image may include at least the fifth assessment location. The ninth image may be associated with the fifth assessment location at a first time index. The tenth image may be associated with the fifth assessment location at a second time index.

The processor may be configured to associate the eleventh image with the twelfth image as a pair. The eleventh image and the twelfth image may include at least the sixth assessment location. The eleventh image may be associated with the sixth assessment location at a first time index. The twelfth image may be associated with the sixth assessment location at a second time index.

The seventh image may include a first set of one or more fourth data channels that may comprise color data and/or topological information corresponding to the fourth assessment location at the first time index. The eighth image may include a second set of the one or more fourth data channels that may comprise color data and topological information corresponding to the fourth assessment location at the second time index.

The ninth image may include a first set of one or more fifth data channels that may comprise color data and/or topological information corresponding to the fifth assessment location at the first time index. The tenth image may include a second set of the one or more fifth data channels that may comprise color data and/or topological information corresponding to the fifth assessment location at the second time index.

The eleventh image may include a first set of one or more sixth data channels that may comprise color data and/or topological information corresponding to the sixth assessment location at the first time index. The twelfth image may include a second set of the one or more sixth data channels that may comprise color data and/or topological information corresponding to the sixth assessment location at the second time index.

In one or more scenarios, the processor may be configured such that the fourth assessment location is at least one of: proximate to the second side of the first tooth along with the fifth assessment location and the sixth assessment location; proximate to the second side of the first tooth along with the fifth assessment location, where the sixth assessment location may be proximate to the first side of the first tooth; and/or proximate to the second side of the first tooth, where the fifth assessment location and the sixth assessment location may be proximate to the first side of the first tooth.

In one or more scenarios, the processor may be configured such that the visually interpretable format for the indication of the first differential MGI value (e.g., a first differential modified gingival index (MGI) value, a first differential gingival index (GI), and/or another first differential index related to a gingival condition) may comprise a numerical representation of the first differential MGI value (e.g., a first differential modified gingival index (MGI) value, a first differential gingival index (GI), and/or another first differential index related to a gingival condition) that may be imposed proximate to the first assessment location on the digital representation of the at least part of the oral cavity.

The processor may be configured such that the visually interpretable format for the indication of the first differential MGI value may comprise a representation of bleeding intensity corresponding to the first differential MGI value that may be imposed proximate to the first assessment location on the digital representation of the at least part of the oral cavity. The bleeding intensity representation may vary from significant bleeding corresponding to a first differential MGI value indication (e.g., a first differential modified gingival index (MGI) value indication, a first differential gingival index (GI) value indication, and/or another first differential index value indication related to a gingival condition) of at least one of: a relatively severe gingivitis condition, or a relatively significantly increased gingivitis condition, to a non-bleeding condition corresponding to a first differential MGI value indication (e.g., a first differential modified gingival index (MGI) value indication, a first differential gingival index (GI) value indication, and/or another first differential index value indication related to a gingival condition) of at least one of: a relatively absent gingivitis condition, or a relatively significantly decreased gingivitis condition.

The processor may be configured such that the visually interpretable format for the indication of the first differential MGI value (e.g., a first differential modified gingival index (MGI) value, a first differential gingival index (GI) value, and/or another first differential index value related to a gingival condition) may comprise a heat map shading differentiation corresponding to the first differential MGI value (e.g., a first differential modified gingival index (MGI) value, a first differential gingival index (GI) value, and/or another first differential index value related to a gingival condition) that may be imposed proximate to the first assessment location on the digital representation of the at least part of the oral cavity. The heat map color differentiation may vary from dark shading corresponding to a first differential MGI value indication (e.g., a first differential modified gingival index (MGI) value indication, a first differential gingival index (GI) value indication, and/or another first differential index value indication related to a gingival condition) of at least one of: a relatively severe gingivitis condition, or a relatively significantly increased gingivitis condition, to a light shading corresponding to a first differential MGI value indication (e.g., a first differential modified gingival index (MGI) value indication, a first differential gingival index (GI) value indication, and/or another first differential index value indication related to a gingival condition) of at least one of: a relatively absent gingivitis condition, or a relatively significantly decreased gingivitis condition.

In one or more scenarios, the processor may be configured to determine, perhaps via the one or more machine-learning algorithms, a second differential MGI value (e.g., a second differential modified gingival index (MGI) value, a second differential gingival index (GI) value, and/or another second differential index value related to a gingival condition) for the second assessment location based, at least in part, on the third image, the fourth image, the first set of the one or more second data channels, and/or the second set of the one or more second data channels.

In one or more scenarios, the processor may be configured to determine, perhaps via the one or more machine-learning algorithms, a third differential MGI value (e.g., a third differential modified gingival index (MGI) value, a third differential gingival index (GI) value, and/or another third differential index value related to a gingival condition) for the third assessment location based, at least in part, on the fifth image, the sixth image, the first set of the one or more third data channels, and/or the second set of the one or more third data channels.

In one or more scenarios, the processor may be configured to determine, perhaps via the one or more machine-learning algorithms, a fourth differential MGI value (e.g., a fourth differential modified gingival index (MGI) value, a fourth differential gingival index (GI) value, and/or another fourth differential index value related to a gingival condition) for the fourth assessment location based, at least in part, on the seventh image, the eight image, the first set of the one or more fourth data channels, and/or the second set of the one or more fourth data channels.

In one or more scenarios, the processor may be configured to determine, perhaps via the one or more machine-learning algorithms, a fifth differential MGI value (e.g., a fifth differential modified gingival index (MGI) value, a fifth differential gingival index (GI) value, and/or another fifth differential index value related to a gingival condition) for the fifth assessment location based, at least in part, on the ninth image, the tenth image, the first set of the one or more fifth data channels, and/or the second set of the one or more fifth data channels.

In one or more scenarios, the processor may be configured to determine, perhaps via the one or more machine-learning algorithms, a sixth differential MGI value (e.g., a sixth differential modified gingival index (MGI) value, a sixth differential gingival index (GI) value, and/or another sixth differential index value related to a gingival condition) for the sixth assessment location based, at least in part, on the eleventh image, the twelfth image, the first set of the one or more sixth data channels, and/or the second set of the one or more sixth data channels.

The processor may be configured to provide an indication of at least one of: the second differential MGI value, the third differential MGI value, the fourth differential MGI value, the fifth differential MGI value, and/or the sixth differential MGI value, in a visually interpretable format via the digital representation of at least a part of the oral cavity on the display device.

In one or more scenarios, the processor may be configured to provide an indication of one or more of: the second differential MGI value, the third differential MGI value, the fourth differential MGI value, the fifth differential MGI value, and/or the sixth differential MGI value in a visually interpretable format via the digital representation of at least a part of the oral cavity on the display device, via a numerical representation of at least one of: the second differential MGI value, the third differential MGI value, the fourth differential MGI value, the fifth differential MGI value, and/or the sixth differential MGI value that may be imposed proximate to the assessment location corresponding to the respective differential MGI value on the digital representation of the at least part of the oral cavity.

In one or more scenarios, the processor may be configured to provide an indication of one or more of: the second differential MGI value, the third differential MGI value, the fourth differential MGI value, the fifth differential MGI value, and/or the sixth differential MGI value in a visually interpretable format via the digital representation of at least a part of the oral cavity on the display device, via a representation of bleeding intensity corresponding to one or more of: the second differential MGI value, the third differential MGI value, the fourth differential MGI value, the fifth differential MGI value, and/or the sixth differential MGI value, that may be imposed proximate to the assessment location corresponding to the respective differential MGI value on the digital representation of the at least part of the oral cavity. The bleeding intensity representation may vary from significant bleeding corresponding to a respective differential MGI value indication of at least one of: a relatively severe gingivitis condition, or a relatively significantly increased gingivitis condition, to a non-bleeding condition corresponding to a respective differential MGI value indication of at least one of: a relatively absent gingivitis condition, or a relatively significantly decreased gingivitis condition.

In one or more scenarios, the processor may be configured to provide an indication of one or more of: the second differential MGI value, the third differential MGI value, the fourth differential MGI value, the fifth differential MGI value, and/or the sixth differential MGI value in a visually interpretable format via the digital representation of at least a part of the oral cavity on the display device, via a heat map shading differentiation corresponding to one or more of: the second differential MGI value, the third differential MGI value, the fourth differential MGI value, the fifth differential MGI value, and/or the sixth differential MGI value, that may be impose proximate to the assessment location corresponding to the respective differential MGI value on the digital representation of the at least part of the oral cavity. The heat map color differentiation for the respective differential MGI values may vary from dark shading corresponding to a relatively high differential MGI value to a light shading corresponding to a relatively low differential MGI value.

In one or more scenarios, the processor may be configured such that the differential MGI value is determined as an integer value from a range of -4 to 4.

In one or more scenarios, the processor may be configured such that the second image may correspond to a second scan of the plurality of scans that is subsequent in time to a first scan of the plurality of scans corresponding to the first image. In one or more scenarios, the period of time is measured in a range of at least one of: days, weeks, or months.

In one or more scenarios, the processor may be configured such that the one or more machine-learning algorithms are adjustable into one or more modes. The one or mode modes may comprise at least an operational mode and a learning mode. The processor may be configured to receive a first calibration image and a second calibration image of a first assessment location of oral cavity. The first calibration image may be associated with the first assessment location at a first time index. The second calibration image may be associated with the first assessment location at a second time index. The first calibration image may include a first set of one or more first calibration data channels. The first set of one or more first calibration data channels may comprise color data and/or topological information corresponding to the first assessment location at the first time index. The second calibration image may include a second set of one or more first calibration data channels that may comprise color data and/or topological information corresponding to the first assessment location at the second time index.

The processor may be configured to associate the first calibration image and the second calibration image as a first calibration image pair. The processor may be configured to receive a first predetermined MGI value (e.g., a first predetermined differential modified gingival index (MGI) value, a first predetermined differential gingival index (GI) value, and/or another first predetermined differential index value related to a gingival condition) for the first calibration image. The processor may be configured to receive a second predetermined MGI value for the second calibration image. The processor may be configured to adjust the one or more machine-learning algorithms into the learning mode.

The processor may be configured to associate, perhaps by the one or more machine-learning algorithms in the learning mode, the first predetermined MGI value (e.g., a first predetermined differential modified gingival index (MGI) value, a first predetermined differential gingival index (GI) value, and/or another first predetermined differential index value related to a gingival condition) with the first calibration image and/or the first set of one or more first calibration data channels. The processor may be configured to associate, perhaps by the one or more machine-learning algorithms in the learning mode, the second predetermined MGI value (e.g., a second predetermined differential modified gingival index (MGI) value, a second predetermined differential gingival index (GI) value, and/or another second predetermined differential index value related to a gingival condition) with the second calibration image and/or the second set of one or more first calibration data channels.

The processor may be configured to perform a calibration on the one or more machine-learning algorithms based, at least in part, on the association of the first predetermined MGI value (e.g., a first predetermined differential modified gingival index (MGI) value, a first predetermined differential gingival index (GI) value, and/or another first predetermined differential index value related to a gingival condition) with the first calibration image and/or the first set of one or more first calibration data channels, and/or the association of the second predetermined MGI value (e.g., a second predetermined differential modified gingival index (MGI) value, a second predetermined differential gingival index (GI) value, and/or another second predetermined differential index value related to a gingival condition) with the second calibration image and/or the second set of one or more first calibration data channels. The calibration may include an iterative update of one or more parameters to minimize at least one of: an objective function, and/or a loss function, of the one or more machine-learning algorithms.

In one or more scenarios, a scanner system, and/or techniques performed thereby, may be configured for detection and/or display of an indication of a periodontal pocket depth of a subject's oral cavity via a digital representation of the oral cavity. The system may comprise a memory, a display device, and/or a processor. The processor may be configured to receive scan data of the oral cavity. The processor may be configured to indicate one or more teeth in the oral cavity based, at least in part, on the scan data. The processor may be configured to indicate a first assessment location proximate to a first tooth of the one or more teeth based, at least in part, on the scan data.

The processor may be configured to generate a first image from the scan data. The first image may include at least the first assessment location and/or one or more first data channels. The one or more first data channels may comprise color data and/or topological information corresponding to the first assessment location.

The processor may be configured to determine, perhaps via one or more machine-learning algorithms, at least one of: a first periodontal pocket depth value, and/or a first periodontal pocket depth value range, for the first assessment location based, at least in part, on the first image and/or the associated one or more first data channels.

The processor may be configured to provide an indication of at least one of: the first periodontal pocket depth value, and/or the first periodontal pocket depth value range for the first assessment location in a visually interpretable format via the digital representation of the oral cavity on the display device.

In one or more scenarios, the topological information may comprise information obtained from the scan data. The topological information may comprise at least one of: one or more facet normals, one or more oral cavity depth measurements, and/or oral cavity surface curvature data.

In one or more scenarios, the scan data may comprise at least a three-dimensional (3D) representation of the oral cavity. In one or more scenarios, the processor may be configured such that the first image may comprise a two-dimensional (2D) image.

In one or more scenarios, the processor may be configured such that the digital representation of the at least part of the oral cavity is at least one of: a two-dimensional (2D) digital representation of the at least part of the oral cavity, and/or a three-dimensional (3D) digital representation of the at least part of the oral cavity.

In one or more scenarios, the system may comprise an intra-oral scanner. The intra-oral scanner may comprise at least one light emitting device. The intra-oral scanner may comprise an imaging sensor. The imaging sensor may be configured to capture reflected light of a dental object in the oral cavity. The imaging sensor may be configured to provide the scan data of the oral cavity, based at least in part, on the reflected light.

In one or more scenarios, the processor may be configured such that the one or more first data channels may comprise at least six first data channels. In one or more scenarios, the processor may be configured such that the color data comprises a color space definition of the first assessment location.

In one or more scenarios, the processor may be configured such that color space definition of the first assessment location may comprise at least one of: a red color, a green color, and/or a blue color. In one or more scenarios, the color data may comprise one or more fluorescence colors.

In one or more scenarios, the processor may be configured such that the one or more facet normals may be communicated as camera image coordinates and/or may comprise one or more of: a Nx component, a Ny component, and/or a Nz component. The processor may be configured such that at least one of: the Nx component, the Ny component, and/or the Nz component may identify the first assessment location in the color space definition of the first assessment location.

In one or more scenarios, the first assessment location may comprise at least some of a gingiva area of the oral cavity. In one or more scenarios, the processor may be configured to determine a tooth number for each of the one or more teeth.

In one or more scenarios, the processor may be configured such that the one or more machine-learning algorithms may determine the at least one of: the first periodontal pocket depth value, and/or the first periodontal pocket depth value range based, at least in part, on one or more of: an outer shape of soft tissue, a gingival color, a shape of a gingival margin, a position of the gingival margin, a position of a cemento-enamel junction (CEJ), and/or a curvature of at least the first assessment location.

In one or more scenarios, the processor may be configured to indicate a first side and a second side of the first tooth, based at least in part, on the scan data. The processor may be configured to indicate a second assessment location proximate to the first tooth, based at least in part, on the scan data. The processor may be configured to indicate a third assessment location proximate to the first tooth, based at least in part, on the scan data.

The processor may be configured to generate a second image from the scan data. The second image may include at least the second assessment location and/or one or more second data channels. The one or more second data channels may comprise color data and/or topological information corresponding to the second assessment location.

The processor may be configured to generate a third image from the scan data. The third image may include at least the third assessment location and/or one or more third data channels. The one or more third data channels may comprise color data and/or topological information corresponding to the third assessment location.

In one or more scenarios, the processor may be configured such that the first assessment location is at least one of: proximate to the first side of the first tooth along with the second assessment location and the third assessment location; proximate to the first side of the first tooth along with the second assessment location, where the third assessment location may be proximate to the second side of the first tooth; and/or proximate to the first side of the first tooth, where the second assessment location and the third assessment location may be proximate to the second side of the first tooth.

In one or more scenarios, the processor may be configured to indicate a fourth assessment location proximate to the first tooth, based at least in part, on the scan data. The processor may be configured to indicate a fifth assessment location proximate to the first tooth, based at least in part, on the scan data. The processor may be configured to indicate a sixth assessment location proximate to the first tooth, based at least in part, on the scan data. The processor may be configured to generate a fourth image from the scan data. The fourth image may include at least the fourth assessment location and/or one or more fourth data channels. The one or more fourth data channels may comprise color data and/or topological information corresponding to the fourth assessment location.

The processor may be configured to generate a fifth image from the scan data. The fifth image may include at least the fifth assessment location and/or one or more fifth data channels. The one or more fifth data channels may comprise color data and/or topological information corresponding to the fifth assessment location. The processor may be configured to generate a sixth image from the scan data. The sixth image may include at least the sixth assessment location and/or one or more sixth data channels. The one or more sixth data channels may comprise color data and/or topological information corresponding to the sixth assessment location.

In one or more scenarios, the processor may be configured such that the fourth assessment location is at least one of: proximate to the second side of the first tooth along with the fifth assessment location and the sixth assessment location; proximate to the second side of the first tooth along with the fifth assessment location, where the sixth assessment location may be proximate to the first side of the first tooth; and/or proximate to the second side of the first tooth, where the fifth assessment location and the sixth assessment location may be proximate to the first side of the first tooth.

In one or more scenarios, the processor may be configured such that the visually interpretable format for the indication of the at least one of: the first periodontal pocket depth value, and/or the first periodontal pocket depth value range, may comprise a numerical representation of the at least one of: the first periodontal pocket depth value, and/or the first periodontal pocket depth value range, that may be imposed proximate to the first assessment location on the digital representation of the at least part of the oral cavity.

In one or more scenarios, the processor may be configured to determine, perhaps via the one or more machine-learning algorithms at least one of: a second periodontal pocket depth value, and/or a second periodontal pocket depth value range for the second assessment location based, at least in part, on the second image and the one or more second data channels. The processor may be configured to determine, perhaps via the one or more machine-learning algorithms, at least one of: a third periodontal pocket depth value, and/or a third periodontal pocket depth value range for the third assessment location based, at least in part, on the third image and the one or more third data channels.

The processor may be configured to determine, perhaps via the one or more machine-learning algorithms, at least one of: a fourth periodontal pocket depth value, and/or a fourth periodontal pocket depth value range for the fourth assessment location based, at least in part, on the fourth image and the one or more fourth data channels. The processor may be configured to determine, perhaps via the one or more machine-learning algorithms, at least one of: a fifth periodontal pocket depth value, and/or a fifth periodontal pocket depth value range for the fifth assessment location based, at least in part, on the fifth image and the one or more fifth data channels.

The processor may be configured to determine, perhaps via the one or more machine-learning algorithms, at least one of: a sixth periodontal pocket depth value, and/or a sixth periodontal pocket depth value range for the sixth assessment location based, at least in part, on the sixth image and the one or more sixth data channels.

The processor may be configured to provide an indication of at least one of: the second periodontal pocket depth value, the second periodontal pocket depth value range, the third periodontal pocket depth value, the third periodontal pocket depth value range, the fourth periodontal pocket depth value, the fourth periodontal pocket depth value range, the fifth periodontal pocket depth value, the fifth periodontal pocket depth value range, the sixth periodontal pocket depth value, and/or the sixth periodontal pocket depth value range, in a visually interpretable format via the digital representation of at least a part of the oral cavity on the display device.

In one or more scenarios, the processor may be configured to provide an indication of one or more of: the second periodontal pocket depth value, the second periodontal pocket depth value range, the third periodontal pocket depth value, the third periodontal pocket depth value range, the fourth periodontal pocket depth value, the fourth periodontal pocket depth value range, the fifth periodontal pocket depth value, the fifth periodontal pocket depth value range, the sixth periodontal pocket depth value, and/or the sixth periodontal pocket depth value range in a visually interpretable format via the digital representation of at least a part of the oral cavity on the display device, via a numerical representation of at least one of: the second periodontal pocket depth value, the second periodontal pocket depth value range, the third periodontal pocket depth value, the third periodontal pocket depth value range, the fourth periodontal pocket depth value, the fourth periodontal pocket depth value range, the fifth periodontal pocket depth value, the fifth periodontal pocket depth value range, the sixth periodontal pocket depth value, and/or the sixth periodontal pocket depth value range, that may be imposed proximate to the assessment location corresponding to the respective periodontal pocket depth value and/or the respective periodontal pocket depth value range on the digital representation of the at least part of the oral cavity.

In one or more scenarios, the processor may be further configured such that the first periodontal pocket depth value may be determined as a real number from a range of 0 to 10 millimeters, and/or the first periodontal pocket depth value range may be at least one of: 0 to 3 millimeters, 3 to 5 millimeters, or greater than 7 millimeters.

In one or more scenarios, the processor may be configured such that the one or more machine-learning algorithms may be adjustable into one or more modes. The one or mode modes may comprise at least an operational mode and a learning mode. The processor may be configured to receive a plurality of calibration images of oral cavity tissue areas. One or more, or each of the plurality of calibration images may include one or more calibration data channels.

The processor may be configured to receive at least one of: a predetermined periodontal pocket depth value, and/or a predetermined periodontal pocket depth value range for one or more, or each of the plurality of calibration images. The processor may be configured to adjust the one or more machine-learning algorithms into the learning mode.

The processor may be configured to associate, perhaps by the one or more machine-learning algorithms in the learning mode, at least one of: the predetermined periodontal pocket depth value, and/or the predetermined periodontal pocket depth value range, for one or more, or each of the plurality of calibration images and/or the corresponding one or more calibration data channels.

The processor may be configured to perform a calibration of the one or more machine-learning algorithms based, at least in part, on the association of the at least one of: the predetermined periodontal pocket depth value, and/or the predetermined periodontal pocket depth value range, with the plurality of calibration images and/or the corresponding one or more calibration data channels. The calibration may include an iterative update of one or more parameters to minimize at least one of: an objective function, and/or a loss function, of the one or more machine-learning algorithms.

In one or more scenarios, a scanner system, and/or techniques that may be performed thereby, may be configured for detection and/or display of an indication of a periodontal pocket depth of a subject's oral cavity via a digital representation of the oral cavity. The system may comprise a memory, a display device, and/or a processor. The processor may be configured to receive scan data of the oral cavity. The scan data may comprise a plurality of scans of the oral cavity obtained from the subject over a period of time. The processor may be configured to indicate one or more teeth in the oral cavity based, at least in part, on the scan data. The processor may be configured to indicate a first assessment location proximate to a first tooth of the one or more teeth, based at least in part, on the scan data.

The processor may be configured to generate at least a first image from the scan data corresponding to a first time index. The first image may include at least the first assessment location at the first time index and/or a first set of one or more first data channels. The first set of one or more first data channels may comprise color data and/or topological information corresponding to the first assessment location at the first time index.

The processor may be configured to generate at least a second image from the scan data corresponding to a second time index. The second image may include at least the second assessment location at the second time index and/or a second set of one or more first data channels. The second set of one or more first data channels may comprise color data and/or topological information corresponding to the first assessment location at the second time index. The processor may be configured to associate the first image with the second image as a pair.

The processor may be configured to determine, perhaps via one or more machine-learning algorithms, a first differential periodontal pocket depth value for the first assessment location based, at least in part, on the first image, the second image, the first set of one or more first data channels, and/or the second set of one or more first data channels. The processor may be configured to provide an indication of the first differential periodontal pocket depth value for the first assessment location in a visually interpretable format via the digital representation of at least a part of the oral cavity on the display device.

In one or more scenarios, the topological information corresponding to the first assessment location at the first time index may comprise information obtained from the scan data. The topological information corresponding to the first assessment location at the first time index may comprise at least one of: one or more facet normal corresponding to the first assessment location at the first time index, one or more oral cavity depth measurements corresponding to the first assessment location at the first time index, and/or oral cavity surface curvature data corresponding to the first assessment location at the first time index.

In one or more scenarios, the topological information corresponding to the first assessment location at the second time index may comprise information obtained from the scan data. The topological information corresponding to the first assessment location at the second time index may comprise at least one of: one or more facet normal corresponding to the first assessment location at the second time index, one or more oral cavity depth measurements corresponding to the first assessment location at the second time index, and/or oral cavity surface curvature data corresponding to the first assessment location at the second time index.

In one or more scenarios, the scan data may comprise at least a three-dimensional (3D) representation of the oral cavity over the period of time. The processor may be configured such that at least one of: the first image, and/or the second image, may comprise a two-dimensional (2D) image.

In one or more scenarios, the processor may be configured such that the digital representation of the at least part of the oral cavity may be at least one of: a two-dimensional (2D) digital representation of the at least part of the oral cavity, and/or a three-dimensional (3D) digital representation of the at least part of the oral cavity.

In one or more scenarios, the system may comprise an intra-oral scanner. The intra-oral scanner may comprise at least one light emitting device. The intra-oral scanner may comprise an imaging sensor. The imaging sensor may be configured to capture reflected light of a dental object in the oral cavity. The imaging sensor may be configured to provide the scan data of the oral cavity, based at least in part, on the reflected light.

In one or more scenarios, the processor may be configured such that the first set of one or more first data channels may comprise a first group of at least six first data channels, and/or the second set of one or more data channels may comprise a second group of at least six first data channels.

In one or more scenarios, the processor may be further configured such that the color data of the first set of one or more first data channels may comprise a color space definition of the first assessment location at the first time index. The color data of the second set of one or more first data channels may comprise a color space definition of the first assessment location at the second time index.

In one or more scenarios, the processor may be configured such that at least one of: the color space definition of the first assessment location at the first time index, and/or the color space definition of the first assessment location at the second time index, may comprise at least one of: a red color, a green color, and/or a blue color.

In one or more scenarios, at least one of: the color data of the first assessment location at the first time index, and/or the color data of the first assessment location at the second time index, may comprise one or more fluorescence colors.

In one or more scenarios, the processor may be configured such that the one or more facet normals associated with the first assessment location at the first time index may be communicated as camera image coordinates and/or may comprise one or more of: a Nx component, a Ny component, and/or a Nz component.

The processor may be configured such that the one or more facet normals associated with the first assessment location at the second time index may be communicated as camera image coordinates and/or may comprise one or more of: a Nx component, a Ny component, and/or a Nz component.

In one or more scenarios, the processor may be configured such that at least one of: the Nx component, the Ny component, and/or the Nz component, corresponding to the first assessment location at the first time index may identify the first assessment location in the color space definition of the first assessment location at the first time index.

The processor may be configured such that at least one of: the Nx component, the Ny component, and/or the Nz component corresponding to the first assessment location at the second time index may identify the first assessment location in the color space definition of the first assessment location at the second time index.

In one or more scenarios, the first assessment location may comprise at least some of a gingiva area of the oral cavity. In one or more scenarios, the processor may be configured to determine a tooth number of each of the one or more teeth.

In one or more scenarios, the processor may be configured such that the one or more machine-learning algorithms may determine the first differential periodontal pocket depth value based, at least in part, on one or more of: an outer shape of soft tissue, a gingival color, a shape of a gingival margin, a position of the gingival margin, a position of a cemento-enamel junction (CEJ), a curvature, a volumetric change in the oral cavity, a shape change in the oral cavity, a surface shape change in the oral cavity, and/or a color change of the soft tissue of the first assessment location.

In one or more scenarios, the processor may be configured to indicate a first side and a second side of the first tooth, based at least in part, on the scan data. The processor may be configured to indicate a second assessment location proximate to the first tooth, based at least in part, on the scan data. The processor may be configured to indicate a third assessment location proximate to the first tooth, based at least in part, on the scan data.

The processor may be configured to generate a third image and a fourth image from the scan data. The processor may be configured to generate a fifth image and a sixth image from the scan data.

The processor may be configured to associate the third image with the fourth image as a pair. The third image and the fourth image may include at least the second assessment location. The third image may be associated with the second assessment location at a first time index. The fourth image may be associated with the second assessment location at a second time index.

The processor may be configured to associate the fifth image with the sixth image as a pair. The fifth image and the sixth image may include at least the third assessment location. The fifth image may be associated with the third assessment location at a first time index. The sixth image may be associated with the third assessment location at a second time index.

The third image may include a first set of one or more second data channels that may comprise color data and/or topological information corresponding to the second assessment location at the first time index. The fourth image may include a second set of the one or more second data channels that may comprise color data and/or topological information corresponding to the second assessment location at the second time index.

The fifth image may include a first set of one or more third data channels that may comprise color data and/or topological information corresponding to the third assessment location at the first time index. The sixth image may include a second set of the one or more third data channels that may comprise color data and/or topological information corresponding to the third assessment location at the second time index.

In one or more scenarios, the processor may be configured such that the first assessment location is at least one of: proximate to the first side of the first tooth along with the second assessment location and the third assessment location; proximate to the first side of the first tooth along with the second assessment location, where the third assessment location may be proximate to the second side of the first tooth; and/or proximate to the first side of the first tooth, where the second assessment location and the third assessment location may be proximate to the second side of the first tooth.

In one or more scenarios, the processor may be configured to indicate a fourth assessment location proximate to the first tooth, based at least in part, on the scan data. The processor may be configured to indicate a fifth assessment location proximate to the first tooth, based at least in part, on the scan data. The processor may be configured to indicate a sixth assessment location proximate to the first tooth, based at least in part, on the scan data.

The processor may be configured to generate a seventh image and an eighth image from the scan data. The processor may be configured to generate a ninth image and a tenth image from the scan data. The processor may be configured to generate an eleventh image and a twelfth image from the scan data.

The processor may be configured to associate the seventh image with the eighth image as a pair. The seventh image and the eighth image may include at least the fourth assessment location. The seventh image may be associated with the fourth assessment location at a first time index. The eighth image may be associated with the fourth assessment location at a second time index.

The processor may be configured to associate the ninth image with the tenth image as a pair. The ninth image and the tenth image may include at least the fifth assessment location. The ninth image may be associated with the fifth assessment location at a first time index. The tenth image may be associated with the fifth assessment location at a second time index.

The processor may be configured to associate the eleventh image with the twelfth image as a pair. The eleventh image and the twelfth image may include at least the sixth assessment location. The eleventh image may be associated with the sixth assessment location at a first time index. The twelfth image may be associated with the sixth assessment location at a second time index.

The seventh image may include a first set of one or more fourth data channels that may comprise color data and/or topological information corresponding to the fourth assessment location at the first time index. The eighth image may include a second set of the one or more fourth data channels that may comprise color data and/or topological information corresponding to the fourth assessment location at the second time index.

The ninth image may include a first set of one or more fifth data channels that may comprise color data and/or topological information corresponding to the fifth assessment location at the first time index. The tenth image may include a second set of the one or more fifth data channels that may comprise color data and/or topological information corresponding to the fifth assessment location at the second time index.

The eleventh image may include a first set of one or more sixth data channels that may comprise color data and/or topological information corresponding to the sixth assessment location at the first time index. The twelfth image may include a second set of the one or more sixth data channels that may comprise color data and/or topological information corresponding to the sixth assessment location at the second time index.

In one or more scenarios, the processor may be configured such that the fourth assessment location may be at least one of: proximate to the second side of the first tooth along with the fifth assessment location and the sixth assessment location; proximate to the second side of the first tooth along with the fifth assessment location, where the sixth assessment location may be proximate to the first side of the first tooth; and/or proximate to the second side of the first tooth, where the fifth assessment location and the sixth assessment location may be proximate to the first side of the first tooth.

In one or more scenarios, the processor may be configured such that the visually interpretable format for the indication of the first differential periodontal pocket depth value, may comprise a numerical representation of the first differential periodontal pocket depth value, that may be imposed proximate to the first assessment location on the digital representation of the at least part of the oral cavity.

In one or more scenarios, the processor may be configured to determine, perhaps via the one or more machine-learning algorithms, a second differential periodontal pocket depth value for the second assessment location based, at least in part, on the third image, the fourth image, the first set of the one or more second data channels, and/or the second set of the one or more second data channels.

The processor may be configured to determine, perhaps via the one or more machine-learning algorithms, a third differential periodontal pocket depth value for the third assessment location based, at least in part, on the fifth image, the sixth image, the first set of the one or more third data channels, and/or the second set of the one or more third data channels.

The processor may be configured to determine, perhaps via the one or more machine-learning algorithms, a fourth differential periodontal pocket depth value for the fourth assessment location based, at least in part, on the seventh image, the eight image, the first set of the one or more fourth data channels, and/or the second set of the one or more fourth data channels.

The processor may be configured to determine, perhaps via the one or more machine-learning algorithms, a fifth differential periodontal pocket depth value for the fifth assessment location based, at least in part, on the ninth image, the tenth image, the first set of the one or more fifth data channels, and/or the second set of the one or more fifth data channels.

The processor may be configured to determine, perhaps via the one or more machine-learning algorithms, a sixth differential periodontal pocket depth value for the sixth assessment location based, at least in part, on the eleventh image, the twelfth image, the first set of the one or more sixth data channels, and/or the second set of the one or more sixth data channels.

The processor may be configured to provide an indication of at least one of: the second differential periodontal pocket depth value, the third differential periodontal pocket depth value, the fourth differential periodontal pocket depth value, the fifth differential periodontal pocket depth value, and/or the sixth differential periodontal pocket depth value, in a visually interpretable format via the digital representation of at least a part of the oral cavity on the display device.

In one or more scenarios, the processor may be configured to provide an indication of one or more of: the second differential periodontal pocket depth value, the third differential periodontal pocket depth value, the fourth differential periodontal pocket depth value, the fifth differential periodontal pocket depth value, and/or the sixth differential periodontal pocket depth value, in a visually interpretable format via the digital representation of at least a part of the oral cavity on the display device, via a numerical representation of at least one of: the second differential periodontal pocket depth value, the third differential periodontal pocket depth value, the fourth differential periodontal pocket depth value, the fifth differential periodontal pocket depth value, and/or the sixth differential periodontal pocket depth value, may be imposed proximate to the assessment location corresponding to the respective differential periodontal pocket depth value on the digital representation of the at least part of the oral cavity.

In one or more scenarios, the processor may be configured such that the first differential periodontal pocket depth value may be determined as a real number from a range of - 10 to 10 millimeters.

In one or more scenarios, the processor may be configured such that the second image corresponds to a second scan of the plurality of scans that may be subsequent in time to a first scan of the plurality of scans corresponding to the first image. In one or more scenarios, the period of time may be measured in a range of at least one of days, weeks, and/or months.

In one or more scenarios, the processor may be configured such that the one or more machine-learning algorithms are adjustable into one or more modes. The one or mode modes may comprise at least an operational mode and a learning mode. The processor may be configured to receive a first calibration image and a second calibration image of a first assessment location of oral cavity. The first calibration image may be associated with the first assessment location at a first time index. The second calibration image may be associated with the first assessment location at a second time index. The first calibration image may include a first set of one or more first calibration data channels. The first set of one or more first calibration data channels may comprise color data and/or topological information corresponding to the first assessment location at the first time index. The second calibration image may include a second set of one or more first calibration data channels that may comprise color data and/or topological information corresponding to the first assessment location at the second time index.

The processor may be configured to associate the first calibration image and the second calibration image as a first calibration image pair. The processor may be configured to receive a first predetermined differential periodontal pocket depth value for the first calibration image. The processor may be configured to receive a second predetermined differential periodontal pocket depth value for the second calibration image. The processor may be configured to adjust the one or more machine-learning algorithms into the learning mode.

The processor may be configured to associate, perhaps by the one or more machine-learning algorithms in the learning mode, the first predetermined differential periodontal pocket depth value with the first calibration image and/or the first set of one or more first calibration data channels.

The processor may be configured to associate, perhaps by the one or more machine-learning algorithms in the learning mode, the second predetermined differential periodontal pocket depth value with the second calibration image and/or the second set of one or more first calibration data channels.

The processor may be configured to perform a calibration of the one or more machine-learning algorithms based, at least in part, on the association of the first predetermined differential periodontal pocket depth value with the first calibration image and/or the first set of one or more first calibration data channels.

The processor may be configured to perform a calibration of the one or more machine-learning algorithms based, at least in part, on the association of the second predetermined differential periodontal pocket depth value with the second calibration image and/or the second set of one or more first calibration data channels. The calibration may include an iterative update of one or more parameters to minimize at least one of: an objective function, and/or a loss function, of the one or more machine-learning algorithms.

In one or more scenarios, one or more methods/techniques, and/or devices implementing such methods/techniques, may be executed for generating training data for a device that may be configured to detect and/or display an indication of a gingivitis condition of a diagnostic subject's oral cavity via a digital representation of the diagnostic subject's oral cavity. One or more method may be performed by at least one computer processing device. One or more methods may comprise receiving scan data of a test subject's oral cavity. One or more methods may comprise indicating one or more teeth in the test subject's oral cavity based, at least in part, on the scan data.

One or more methods may comprise indicating a plurality of assessment locations, based at least in part, on the scan data. One or more, or each of the plurality of assessment locations may be proximate to a tooth of the one or more teeth. One or more methods may comprise generating a plurality of calibration images from the scan data. One or more, or each of the plurality of calibration images may include at least one assessment location of the plurality of assessment locations and/or at least one set of one or more data channels. One or more, or each of the at least one set of one or more data channels may comprise color data and/or topological information associated with the at least one assessment location.

One or more methods may comprise receiving a respective predetermined modified gingival index (MGI) (e.g., a respective predetermined modified gingival index (MGI) value, a respective predetermined gingival index (GI) value, and/or another respective predetermined index value related to a gingival condition) for each of the plurality of calibration images. One or more methods may comprise associating each of the respective predetermined MGI with each of the calibration images and each of the at least one set of one or more data channels to form an MGI calibration data set (e.g., a modified gingival index (MGI) calibration data set, a gingival index (GI) calibration data set, and/or another index calibration data set related to a gingival condition) for one or more machine-learning algorithms. One or more methods may comprise storing the MGI calibration data set (e.g., a modified gingival index (MGI) calibration data set, a gingival index (GI) calibration data set, and/or another index calibration data set related to a gingival condition) into a memory of the computer processing device.

In one or more scenarios, the color data of each of the at least one set of one or more data channels may comprise a respective color space definition of the at least one assessment location included in the calibration image. In one or more scenarios, each of the respective color space definitions comprises at least one of: a red color, a green color, and/or a blue color. In one or more scenarios, the color data may comprise one or more fluorescence colors.

In one or more scenarios, one or more methods/techniques, and/or devices implementing such methods/techniques, may be executed for generating training data for a device configured to detect and/or display an indication of a periodontal pocket depth value of a diagnostic subject's oral cavity via a digital representation of the diagnostic subject's oral cavity. One or more methods may be performed by at least one computer processing device. One or more methods may comprise receiving scan data of a test subject's oral cavity. One or more methods may comprise indicating one or more teeth in the test subject's oral cavity based, at least in part, on the scan data. One or more methods may comprise indicating a plurality of assessment locations, based at least in part, on the scan data, each of the plurality of assessment locations proximate to a tooth of the one or more teeth.

One or more methods may comprise generating a plurality of calibration images from the scan data. One or more, or each of the plurality of calibration images may include at least one assessment location of the plurality of assessment locations and/or at least one set of one or more data channels. One or more, or each of the at least one set of one or more data channels may comprise color data and/or topological information associated with the at least one assessment location.

One or more methods may comprise receiving at least one of: a respective predetermined periodontal pocket depth value, and/or a respective predetermined periodontal pocket depth value range, for each of the plurality of calibration images.

One or more methods may comprise associating at least one of: each of the respective predetermined periodontal pocket depth value, and/or each of the respective predetermined periodontal pocket depth value range, with each of the calibration images and each of the at least one set of one or more data channels to form a periodontal pocket depth calibration data set for one or more machine-learning algorithms. One or more methods may comprise storing the periodontal pocket depth calibration data set into a memory of the computer processing device.

In one or more scenarios, the color data of each of the at least one set of one or more data channels may comprise a respective color space definition of the at least one assessment location included in the calibration image.

In one or more scenarios, each of the respective color space definitions comprises at least one of: a red color, a green color, and/or a blue color. In one or more scenarios, the color data may comprise one or more fluorescence colors.

In one or more scenarios, one or more methods/techniques, and/or devices implementing such methods/techniques, may be executed for generating diagnostic data for a device that may be configured to detect and/or display an indication of a gingivitis condition of a subject's oral cavity via a digital representation of the subject's oral cavity. One or more methods may be performed by at least one computer processing device. One or more methods may comprise receiving scan data of the subject's oral cavity. One or more methods may comprise indicating one or more teeth in the subject's oral cavity based, at least in part, on the scan data.

One or more methods may comprise indicating a plurality of assessment locations, based at least in part, on the scan data. One or more, or each of the plurality of assessment locations may be proximate to a tooth of the one or more teeth.

One or more methods may comprise generating a plurality of diagnostic images from the scan data. One or more, or each of the plurality of diagnostic images may include at least one assessment location of the plurality of assessment locations and/or at least one set of one or more data channels. One or more, or each of the at least one set of one or more data channels may comprise color data and/or topological information associated with the at least one assessment location.

One or more methods may comprise associating each of the diagnostic images and each of the at least one set of one or more data channels to form a modified gingival index (MGI) diagnostic data set (e.g., a modified gingival index (MGI) data set, a gingival index (GI) data set, and/or another index data set related to a gingival condition) for one or more machine-learning algorithms. One or more methods may comprise storing the MGI diagnostic data set (e.g., a modified gingival index (MGI) data set, a gingival index (GI) data set, and/or another index data set related to a gingival condition) into a memory of the computer processing device.

In one or more scenarios, the color data of each of the respective at least one set of one or more data channels may comprise a respective color space definition of the at least one assessment location included in the associated diagnostic image. In one or more scenarios, each of the respective color space definitions may comprise at least one of: a red color, a green color, and/or a blue color.

In one or more scenarios, one or more methods/techniques, and/or devices implementing such methods/techniques, may be executed for generating diagnostic data for a device that may be configured to detect and/or display an indication of a periodontal pocket depth value of a subject's oral cavity via a digital representation of the subject's oral cavity. One or more methods may be performed by at least one computer processing device. One or more methods may comprise receiving scan data of the subject's oral cavity. One or more methods may comprise indicating one or more teeth in the test subject's oral cavity based, at least in part, on the scan data. One or more methods may comprise indicating a plurality of assessment locations, based at least in part, on the scan data. One or more, or each of the plurality of assessment locations may be proximate to a tooth of the one or more teeth.

One or more methods may comprise generating a plurality of diagnostic images from the scan data. One or more, or each of the plurality of diagnostic images may include at least one assessment location of the plurality of assessment locations and/or at least one set of one or more data channels. One or more, or each of the at least one set of one or more data channels may comprise color data and/or topological information that may be associated with the at least one assessment location included in the diagnostic image.

One or more methods may comprise associating each of the diagnostic images and each of the at least one set of one or more data channels to form a periodontal pocket depth diagnostic data set for one or more machine-learning algorithms. One or more methods may comprise storing the periodontal pocket depth diagnostic data set into a memory of the computer processing device.

In one or more scenarios, the color data of each of the respective at least one set of one or more data channels may comprise a respective color space definition of the at least one assessment location included in the diagnostic image. In one or more scenarios, each of the respective color space definitions may comprise at least one of: a red color, a green color, and/or a blue color.

One or more non-transitory computer readable mediums may have instructions stored thereon. The instructions may cause at least one processor of an imaging analysis device to perform one or more operations. The imaging analysis device may be in communication with a display device. The one or more operations may comprise receiving scan data of a subject's oral cavity. The one or more operations may comprise indicating one or more teeth in the oral cavity based, at least in part, on the scan data. The one or more operations may comprise indicating a first assessment location that may be proximate to a first tooth of the one or more teeth, based at least in part, on the scan data.

The one or more operations may comprise generating a first image from the scan data. The first image may include at least the first assessment location and/or one or more first data channels. The one or more first data channels may comprise color data and/or topological information corresponding to the first assessment location.

The one or more operations may comprise determining, perhaps via one or more machine-learning algorithms, a first modified gingival index (MGI) value (e.g., a first modified gingival index (MGI) value, a first gingival index (GI) value, and/or another first index value related to a gingival condition) for the first assessment location based, at least in part, on the first image and the one or more first data channels. The one or more operations may comprise providing an indication of the first MGI value (e.g., a first modified gingival index (MGI) value, a first gingival index (GI) value, and/or another first index value related to a gingival condition) in a visually interpretable format via a digital representation of at least a part of the oral cavity on the display device.

One or more non-transitory computer readable mediums may have instructions stored thereon. The instructions may cause at least one processor of an imaging analysis device to perform one or more operations. The imaging analysis device may be in communication with a display device. The one or more operations may comprise receiving scan data of a subject's oral cavity. The scan data may comprise a plurality of scans of the oral cavity obtained from the subject over a period of time. The one or more operations may comprise indicating one or more teeth in the oral cavity based, at least in part, on the scan data. The one or more operations may comprise indicating a first assessment location that may be proximate to a first tooth of the one or more teeth, based at least in part, on the scan data.

The one or more operations may comprise generating at least a first image from the scan data corresponding to a first time index. The first image may include at least the first assessment location at the first time index and/or a first set of one or more first data channels. The first set of one or more first data channels may comprise color data and/or topological information corresponding to the first assessment location at the first time index.

The one or more operations may comprise generating at least a second image from the scan data corresponding to a second time index. The second image may include at least the second assessment location at the second time index and/or a second set of one or more first data channels. The second set of one or more first data channels may comprise color data and/or topological information corresponding to the first assessment location at the second time index. The one or more operations may comprise associating a first image with a second image as a pair.

The one or more operations may comprise determining, perhaps via one or more machine-learning algorithms, a first differential modified gingival index (MGI) value (e.g., a first differential modified gingival index (MGI) value, a first differential gingival index (GI) value, and/or another first differential index value related to a gingival condition) for first assessment location, based, at least in part, on the first image, the second image, the first set of one or more first data channels, and/or the second set of one or more first data channels.

The one or more operations may comprise providing an indication of the first differential MGI value (e.g., a first differential modified gingival index (MGI) value, a first differential gingival index (GI) value, and/or another first differential index value related to a gingival condition) for the first assessment location in a visually interpretable format via a digital representation of at least a part of the oral cavity on the display device.

One or more non-transitory computer readable mediums may have instructions stored thereon. The instructions may cause at least one processor of an imaging analysis device to perform one or more operations. The imaging analysis device may be in communication with a display device. The one or more operations may comprise receiving scan data of a subject's oral cavity. The one or more operations may comprise indicating one or more teeth in the oral cavity based, at least in part, on the scan data. The one or more operations may comprise indicating a first assessment location proximate to a first tooth of the one or more teeth based, at least in part, on the scan data.

The one or more operations may comprise generating a first image from the scan data. The first image may include at least the first assessment location and/or one or more first data channels. The one or more first data channels may comprise color data and/or topological information corresponding to the first assessment location.

The one or more operations may comprise determining, perhaps via one or more machine-learning algorithms, at least one of: a first periodontal pocket depth value, and/or a first periodontal pocket depth value range, for the first assessment location based, at least in part, on the first image and the one or more first data channels. The one or more operations may comprise providing an indication of at least one of: the first periodontal pocket depth value, and/or the first periodontal pocket depth value range for the first assessment location in a visually interpretable format via a digital representation of the oral cavity on the display device.

Those skilled in the art will appreciate that the subject matter described herein may at least facilitate a more efficient, a more uniform, a more accurate, and/or a more useful assessment/measurement of a dental patient's oral cavity gingivitis condition and/or periodontal pocket depth(s). For example, perhaps instead of having a human dental clinician physically examine a dental patient's oral cavity so that the human dental clinician may assess the patient's gingivitis condition and/or to measure periodontal pocket depth(s), the systems/devices and/or techniques described herein may provide for a more efficient and/or a more uniform (e.g., referenced to objective standards, among various dental patients, among the same dental patient over time, etc.) assessment/measurement of the patient's gingivitis condition and/or periodontal pocket depth(s). The systems/devices and/or techniques described herein may offer a more useful and/or a more accurate assessment/measurement of a dental patient's gingivitis condition and/or periodontal pocket depth(s) such that helpful therapeutic intervention may be conducted in a more timely fashion. More timely interventions to treat the dental patient's gingivitis and/or periodontitis may increase the possibility of better treatment outcomes/success.

Without the capabilities of the systems/devices and/or techniques described herein, the ordinarily skilled artisan would not appreciate how such more efficient, more uniform, more accurate, and/or more useful assessment/measurement of a dental patient's oral cavity gingivitis condition and/or periodontal pocket depth(s) may be obtained using 3D scan data of the patient's oral cavity (e.g., 3D scan data from an intra-oral scanner).

One or more non-transitory computer readable mediums may have instructions stored thereon. The instructions may cause at least one processor of an imaging analysis device to perform one or more operations. The imaging analysis device may be in communication with a display device. The one or more operations may comprise receiving scan data of a subject's oral cavity. The scan data may comprise a plurality of scans of the oral cavity obtained from the subject over a period of time. The one or more operations may comprise indicating one or more teeth in the oral cavity based, at least in part, on the scan data. The one or more operations may comprise indicating a first assessment location proximate to a first tooth of the one or more teeth, based at least in part, on the scan data.

The one or more operations may comprise generating at least a first image from the scan data corresponding to a first time index. The first image may include at least the first assessment location at the first time index and/or a first set of one or more first data channels. The first set of one or more first data channels may comprise color data and/or topological information corresponding to the first assessment location at the first time index.

The one or more operations may comprise generating at least a second image from the scan data corresponding to a second time index. The second image may include at least the second assessment location at the second time index and/or a second set of one or more first data channels. The second set of one or more first data channels may comprise color data and/or topological information corresponding to the first assessment location at the second time index. The one or more operations may comprise associating a first image with a second image as a pair.

The one or more operations may comprise determining, perhaps via one or more machine-learning algorithms, a first differential periodontal pocket depth value for the first assessment location based, at least in part, on the first image, the second image, the first set of one or more first data channels, and/or the second set of one or more first data channels.

The one or more operations may comprise providing an indication of the first differential periodontal pocket depth value for the first assessment location in a visually interpretable format via a digital representation of at least a part of the oral cavity on the display device.

The subject matter of this disclosure, and components thereof, can be realized by instructions that upon execution cause one or more processing devices to carry out the processes and/or functions described herein. Such instructions can, for example, comprise interpreted instructions, such as script instructions, e.g., JavaScript or ECMAScript instructions, or executable code, and/or other instructions stored in a computer readable medium.

Implementations of the subject matter and/or the functional operations described in this specification and/or the accompanying figures can be provided in digital electronic circuitry, in computer software, firmware, and/or hardware, including the structures disclosed in this specification and their structural equivalents, and/or in combinations of one or more of them. The subject matter described in this specification can be implemented as one or more computer program products, *e.g.*, one or more modules of computer program instructions encoded on a tangible program carrier for execution by, and/or to control the operation of, data processing apparatus.

A computer program (also known as a program, software, software application, script, or code) can be written in any form of programming language, including compiled or interpreted languages, and/or declarative or procedural languages. It can be deployed in any form, including as a stand-alone program or as a module, component, subroutine, and/or other unit suitable for use in a computing environment. A computer program may or might not correspond to a file in a file system. A program can be stored in a portion of a file that holds other programs and/or data (*e.g.,* one or more scripts stored in a markup language document), in a single file dedicated to the program in question, and/or in multiple coordinated files (*e.g.,* files that store one or more modules, sub programs, or portions of code). A computer program can be deployed to be executed on one computer or on multiple computers that may be located at one site or distributed across multiple sites and/or interconnected by a communication network.

The processes and/or logic flows described in this specification and/or in the accompanying figures may be performed by one or more programmable processors executing one or more computer programs to perform functions by operating on input data and/or generating output, thereby tying the process to a particular machine (*e.g.*, a machine programmed to perform the processes described herein). The processes and/or logic flows can also be performed by, and apparatus can also be implemented as, special purpose logic circuitry, *e.g.*, an FPGA (field programmable gate array) and/or an ASIC (application specific integrated circuit).

Computer readable media suitable for storing computer program instructions and/or data may include all forms of non-volatile memory and/or non-transitory media and memory devices, including by way of example semiconductor memory devices (*e.g.,* EPROM, EEPROM, and/or flash memory devices); magnetic disks (*e.g.,* internal hard disks or removable disks); magneto optical disks; and/or CD ROM and DVD ROM disks. The processor and/or the memory can be supplemented by, or incorporated in, special purpose logic circuitry.

While this specification and the accompanying figures contain many specific implementation details, these should not be construed as limitations on the scope of any invention and/or of what may be claimed, but rather as descriptions of features that may be specific to described example implementations. Certain features that are described in this specification in the context of separate implementations can also be implemented in combination in perhaps one implementation. Various features that are described in the context of perhaps one implementation can also be implemented in multiple combinations separately or in any suitable sub-combination. Although features may be described above as acting in certain combinations and/or perhaps even (*e.g.,* initially) claimed as such, one or more features from a claimed combination can in some cases be excised from the combination. The claimed combination may be directed to a sub-combination and/or variation of a sub-combination.

While operations may be depicted in the drawings in an order, this should not be understood as requiring that such operations be performed in the particular order shown and/or in sequential order, and/or that all illustrated operations be performed, to achieve useful outcomes. The described program components and/or systems can generally be integrated together in a single software product and/or packaged into multiple software products.

Examples of the subject matter described in this specification have been described. The actions recited in the claims can be performed in a different order and still achieve useful outcomes, unless expressly noted otherwise. For example, the processes depicted in the accompanying figures do not require the particular order shown, and/or sequential order, to achieve useful outcomes. Multitasking and parallel processing may be advantageous in one or more scenarios.

While the present disclosure has been illustrated and described in detail in the drawings and foregoing description, the same is to be considered as illustrative and not restrictive in character, it being understood that only certain examples have been shown and described, and that all changes and modifications that come within the spirit of the present disclosure are desired to be protected.

## Claims

1. A scanner system configured for detection and display of an indication of a gingivitis condition of a subject's oral cavity via a digital representation of the oral cavity, the system comprising:
a memory;
a display device; and
a processor, the processor configured at least to:
receive scan data of the oral cavity, wherein the scan data comprises a three-dimensional (3D) representation of the oral cavity;
indicate one or more teeth in the oral cavity based, at least in part, on the scan data;
indicate a first assessment location proximate to a first tooth of the one or more teeth, based at least in part, on the scan data;
generate a first image from the scan data, the first image including at least the first assessment location and one or more first data channels, the one or more first data channels comprising color data and topological information corresponding to the first assessment location;
determine, via one or more machine-learning algorithms, a first modified gingival index (MGI) value for the first assessment location based, at least in part, on the first image and the one or more first data channels; and
provide an indication of the first MGI value in a visually interpretable format via the digital representation of at least a part of the oral cavity on the display device.

2. The system of claim 1, wherein the topological information comprises information obtained from the scan data, the topological information comprising at least one of: one or more facet normals, one or more oral cavity depth measurements, or oral cavity surface curvature data.

3. The system of any of the preceding claims, wherein the processor is further configured such that the first image comprises a two-dimensional (2D) image.

4. The system of any of the preceding claims, wherein the processor is further configured such that the one or more first data channels comprise at least six first data channels.

5. The system of claim 2, wherein the processor is further configured such that the color data comprises a color space definition of the first assessment location.

6. The system of claim 5, wherein the processor is further configured such that the color space definition of the first assessment location comprises at least one of: a red color, a green color, or a blue color.

7. The system of claim 5, wherein the processor is further configured such that the one or more facet normals are communicated as camera image coordinates and comprise one or more of: a Nx component, a Ny component, or a Nz component.

8. The system of claim 7, wherein the processor is further configured such that at least one of: the Nx component, the Ny component, or the Nz component identify the first assessment location in the color space definition of the first assessment location.

9. The system of any of the preceding claims, wherein the first assessment location comprises at least some of a gingiva area of the oral cavity.

10. The system of any of the proceeding claims, wherein the processor is further configured such that the one or more machine-learning algorithms determine the first MGI value based, at least in part, on one or more of: an outer shape of soft tissue, a gingival color, a shape of a gingival margin, a position of the gingival margin, a position of a cemento-enamel junction (CEJ), or a curvature of at least the first assessment location.

11. The system of any of the preceding claims, wherein the processor is further configured to:
indicate a first side and a second side of the first tooth, based at least in part, on the scan data;
indicate a second assessment location proximate to the first tooth, based at least in part, on the scan data;
indicate a third assessment location proximate to the first tooth, based at least in part, on the scan data;
generate a second image from the scan data, the second image including at least the second assessment location and one or more second data channels, the one or more second data channels comprising color data and topological information corresponding to the second assessment location; and
generate a third image from the scan data, the third image including at least the third assessment location and one or more third data channels, the one or more third data channels comprising color data and topological information corresponding to the third assessment location.

12. The system of claim 11, wherein the processor is further configured such that the first assessment location is at least one of: proximate to the first side of the first tooth along with the second assessment location and the third assessment location; proximate to the first side of the first tooth along with the second assessment location, the third assessment location being proximate to the second side of the first tooth; or proximate to the first side of the first tooth, the second assessment location and the third assessment location being proximate to the second side of the first tooth.

13. The system of claim 11, wherein the processor is further configured to:
indicate a fourth assessment location proximate to the first tooth, based at least in part, on the scan data;
indicate a fifth assessment location proximate to the first tooth, based at least in part, on the scan data;
indicate a sixth assessment location proximate to the first tooth, based at least in part, on the scan data;
generate a fourth image from the scan data, the fourth image including at least the fourth assessment location and one or more fourth data channels, the one or more fourth data channels comprising color data and topological information corresponding to the fourth assessment location;
generate a fifth image from the scan data, the fifth image including at least the fifth assessment location and one or more fifth data channels, the one or more fifth data channels comprising color data and topological information corresponding to the fifth assessment location; and
generate a sixth image from the scan data, the sixth image including at least the sixth assessment location and one or more sixth data channels, the one or more sixth data channels comprising color data and topological information corresponding to the sixth assessment location.

14. The system of claim 13, wherein the processor is further configured such that the fourth assessment location is at least one of: proximate to the second side of the first tooth along with the fifth assessment location and the sixth assessment location; proximate to the second side of the first tooth along with the fifth assessment location, the sixth assessment location being proximate to the first side of the first tooth; or proximate to the second side of the first tooth, the fifth assessment location and the sixth assessment location being proximate to the first side of the first tooth.

15. The system of claim 1, wherein the processor is further configured such that the visually interpretable format for the indication of the first MGI value comprises at least one of:
a numerical representation of the first MGI value imposed proximate to the first assessment location on the digital representation of the at least part of the oral cavity;
a representation of bleeding intensity corresponding to the first MGI value imposed proximate to the first assessment location on the digital representation of the at least part of the oral cavity, the bleeding intensity representation varying from significant bleeding corresponding to a relatively high first MGI value to a non-bleeding condition corresponding to a relatively low first MGI value; or
a heat map shading differentiation corresponding to the first MGI value imposed proximate to the first assessment location on the digital representation of the at least part of the oral cavity, the heat map color differentiation varying from dark shading corresponding to a relatively high first MGI value to a light shading corresponding to a relatively low first MGI value.

16. The system of claim 13, wherein the processor is further configured to:
determine, via the one or more machine-learning algorithms, one or more of:
a second MGI value for the second assessment location based, at least in part, on the second image and the one or more second data channels;
a third MGI value for the third assessment location based, at least in part, on the third image and the one or more third data channels;
a fourth MGI value for the fourth assessment location based, at least in part, on the fourth image and the one or more fourth data channels;
a fifth MGI value for the fifth assessment location based, at least in part, on the fifth image and the one or more fifth data channels;
a sixth MGI value for the sixth assessment location based, at least in part, on the sixth image and the one or more sixth data channels; and
provide an indication of at least one of: the second MGI value, the third MGI value, the fourth MGI value, the fifth MGI value, or the sixth MGI value, in a visually interpretable format via the digital representation of at least a part of the oral cavity on the display device.

17. The system of claim 14, wherein the processor is further configured to provide an indication of one or more of: the second MGI value, the third MGI value, the fourth MGI value, the fifth MGI value, or the sixth MGI value in a visually interpretable format via the digital representation of at least a part of the oral cavity on the display device, via one or more of:
a numerical representation of at least one of: the second MGI value, the third MGI value, the fourth MGI value, the fifth MGI value, or the sixth MGI value imposed proximate to the assessment location corresponding to the respective MGI value on the digital representation of the at least part of the oral cavity;
a representation of bleeding intensity corresponding to one or more of: the second MGI value, the third MGI value, the fourth MGI value, the fifth MGI value, or the sixth MGI value, imposed proximate to the assessment location corresponding to the respective MGI value on the digital representation of the at least part of the oral cavity, the bleeding intensity representation for the respective MGI values varying from significant bleeding corresponding to a relatively high MGI value to a non-bleeding condition corresponding to a relatively low MGI value; or
a heat map shading differentiation corresponding to one or more of: the second MGI value, the third MGI value, the fourth MGI value, the fifth MGI value, or the sixth MGI value, imposed proximate to the assessment location corresponding to the respective MGI value on the digital representation of the at least part of the oral cavity, the heat map color differentiation for the respective MGI values varying from dark shading corresponding to a relatively high MGI value to a light shading corresponding to a relatively low MGI value.

18. The system of any of the preceding claims, wherein the processor is further configured such that the one or more machine-learning algorithms are adjustable into one or more modes, the one or mode modes comprising at least an operational mode and a learning mode, wherein the processor is further configured to:
receive a plurality of calibration images of oral cavity tissue areas, each of the plurality of calibration images including one or more calibration data channels;
receive a predetermined MGI value for each of the plurality of calibration images;
adjust the one or more machine-learning algorithms into the learning mode;
associate, by the one or more machine-learning algorithms in the learning mode, the predetermined MGI value for each of the plurality of calibration images and the corresponding one or more calibration data channels; and
perform a calibration of the one or more machine-learning algorithms based, at least in part, on the association of the predetermined MGI value with the plurality of calibration images and the corresponding one or more calibration data channels, the calibration including an iterative update of one or more parameters to minimize at least one of: an objective function, or a loss function, of the one or more machine-learning algorithms.

19. A method for generating training data for a device configured to detect and display an indication of a gingivitis condition of a diagnostic subject's oral cavity via a digital representation of the diagnostic subject's oral cavity, the method performed by at least one computer processing device, the method comprising:
receiving scan data of a test subject's oral cavity;
indicating one or more teeth in the test subject's oral cavity based, at least in part, on the scan data;
indicating a plurality of assessment locations, based at least in part, on the scan data, each of the plurality of assessment locations proximate to a tooth of the one or more teeth;
generating a plurality of calibration images from the scan data, each of the plurality of calibration images including at least one assessment location of the plurality of assessment locations and at least one set of one or more data channels, each of the at least one set of one or more data channels comprising color data and topological information associated with the at least one assessment location;
receiving a respective predetermined modified gingival index (MGI) for each of the plurality of calibration images;
associating each of the respective predetermined MGI with each of the calibration images and each of the at least one set of one or more data channels to form an MGI calibration data set for one or more machine-learning algorithms; and
storing the MGI calibration data set into a memory of the computer processing device.

20. A non-transitory computer readable medium having instructions stored thereon, the instructions causing at least one processor of an imaging analysis device to perform one or more operations, the imaging analysis device being in communication with a display device, the one or more operations comprising at least:
receiving scan data of a subject's oral cavity;
indicating one or more teeth in the oral cavity based, at least in part, on the scan data;
indicating a first assessment location proximate to a first tooth of the one or more teeth, based at least in part, on the scan data;
generating a first image from the scan data, the first image including at least the first assessment location and one or more first data channels, the one or more first data channels comprising color data and topological information corresponding to the first assessment location;
determining, via one or more machine-learning algorithms, a first modified gingival index (MGI) value for the first assessment location based, at least in part, on the first image and the one or more first data channels; and
providing an indication of the first MGI value in a visually interpretable format via a digital representation of at least a part of the oral cavity on the display device.

21. A processing device configured for detection and display of an indication of a gingivitis condition of a subject's oral cavity via a digital representation of the oral cavity, the device comprising:
a means for receiving scan data of the oral cavity;
a means for indicating one or more teeth in the oral cavity based, at least in part, on the scan data;
a means for indicating a first assessment location proximate to a first tooth of the one or more teeth, based at least in part, on the scan data;
a means for generating a first image from the scan data, the first image including at least the first assessment location and one or more first data channels, the one or more first data channels comprising color data and topological information corresponding to the first assessment location;
a means for determining a first modified gingival index (MGI) value for the first assessment location based, at least in part, on the first image and the one or more first data channels; and
a means for indicating of the first MGI value in a visually interpretable format via the digital representation of at least a part of the oral cavity.
